# EUROPÄISCHE PATENTSCHRIFT

(11) **EP 1 362 125 B1**
(45) Veröffentlichungstag und Bekanntmachung des Hinweises auf die Patenterteilung: **10.11.2010**
(21) Anmeldenummer: 02724180.1
(22) Anmeldetag: 22.02.2002
(51) Int. Cl.: C12Q 1/68, C12N 15/10, C12N 15/39, C07K 14/07

(54) **VERFAHREN ZUR IDENTIFIZIERUNG BIOLOGISCH AKTIVER STRUKTUREN MIKROBIELLER ERREGER**
METHOD FOR IDENTIFYING of BIOLOGICALLY ACTIVE STRUCTURES OF MICROBIAL PATHOGENS
PROCEDE POUR IDENTIFIER DES STRUCTURES BIOLOGIQUEMENT ACTIVES D'AGENTS PATHOGENES MICROBIENS

(30) Priorität: 22.02.2001 DE 10108626
(43) Veröffentlichungstag der Anmeldung: 19.11.2003
(73) Patentinhaber: Sahin, Ugur, 55131 Mainz (DE); Tuereci, Ozlem, 55131 Mainz (DE); Ludewig, Burkhard, 55131 Mainz (DE)
(72) Erfinder: SAHIN, Ugur c/o III Medizinische Klinik, 55131 Mainz (DE); TUERECI, Özlem c/o III Medizinische Klinik, 55131 Mainz (DE); LUDEWIG, Burkhard c/o Kantonal Hospital St. Gallen, 9007 St. Gallen (CH)
(74) Vertreter: Schnappauf, Georg
(86) Internationale Anmeldenummer: PCT/EP2002/001909
(87) Internationale Veröffentlichungsnummer: WO 2002/068682

(56) Entgegenhaltungen:
- WO-A-90/09457
- US-A- 5 731 171
- LANG SUSAN ET AL: "Identification of a novel antigen from Staphylococcus epidermidis." FEMS IMMUNOLOGY AND MEDICAL MICROBIOLOGY, Bd. 29, Nr. 3, November 2000 (2000-11), Seiten 213-220, XP002225330 ISSN: 0928-8244
- PADMALAYAM INDIRA ET AL: "Molecular cloning, sequencing, expression, and characterization of an immunogenic 43-kilodalton lipoprotein of Bartonella bacilliformis that has homology to NlpD/LppB." INFECTION AND IMMUNITY, Bd. 68, Nr. 9, September 2000 (2000-09), Seiten 4972-4979, XP002225331 ISSN: 0019-9567

## Beschreibung

Die vorliegende Erfindung betrifft ein Verfahren zur Identifizierung biologisch aktiver Strukturen, die durch das Genom von mikrobiellen Erregern kodiert werden, ausgehend von genomischen Erregemukleinsäuren.

### Hintergrund der Erfindung

Die Entwicklung von molekular definierten Serodiagnostika und Impfstoffen setzt die molekulare Kenntnis und die Verfügbarkeit der durch das Immunsystem eines infizierten Wirtes erkannten Antigene des pathogenen Erregers (das mikrobielle Immunom) voraus. Die Serodiagnostik von Infektionskrankheiten basiert auf dem Nachweis von im Blut zirkulierenden Antikörpern, die spezifisch gegen immunogene Bestandteile (Antigene) des Erregers gerichtet sind und somit eine vorhandene oder abgelaufene Infektion anzeigen. Die Kenntnis dieser Antigene erlaubt es, dieselben als molekular definierte Impfstoffe rekombinant herzustellen. Diese Impfstoffe können einem Organismus einen Schutz vor einer Infektion mit dem betreffenden Erreger bieten (prophylaktische Immunisierung) aber auch bei persistierenden und chronischen Erregern zu deren Eliminierung dienen (therapeutische Immunisierung). Die Bedeutung, die solchen Antigenen sowohl für eine spezifische Diagnostik als auch für eine spezifische Therapie zukommt, führt zu einem beträchtlichen Interesse an der Identifikation dieser Strukturen.

Während für niedrigkomplexe Infektionserreger mit bekanntem Genom (z.B. einfache Viren) die meisten relevanten Antigene molekular identifiziert sind, ist noch unklar, welche Antigene bei komplexeren Infektionserregern (z.B. Bakterien) immunologisch relevant sind. Die Ursache hierfür liegt darin, dass die komplexen Genome dieser Erreger eine Vielzahl von Genen (1000 bis über 4000) enthalten, die eine schnelle Identifikation der relevanten Antigene erschweren. Selbst bei Erregern, deren Genome vollständig bekannt sind, muss man davon ausgehen, dass die Zuordnung aller Nukleotidbereiche zu tatsächlich für Proteine kodierenden -und somit potentiell Antigene ergebenden Abschnitten- noch nicht vollständig erfolgt ist.

Eine Reihe von Techniken, die zur Identifikation von Antigenen in den letzten Jahren entwickelt wurden, versuchen dieser Komplexität gerecht zu werden. Die meisten dieser Verfahren stammen aus dem Feld der "Proteomics"-Technologien, welche Hochdurchsatz-Technologien der Proteinanalyse bezeichnet.

Eine dieser Hochdurchsatz-Technologien umfasst die Verwendung von 2-D-Gelen (z.B. Liu B, Marks JD. (2000), Anal. Biochem. 286,1191-28). Da bei diesem Verfahren große Mengen von Erregern benötigt werden, werden diese zunächst unter Kulturbedingungen angezüchtet. Danach werden Extrakte aus lysierten Erregern hergestellt und die darin befindlichen Proteine per Gelelektrophorese aufgetrennt. Durch Immunseren (Patientenseren, Seren immunisierter Tiere) erkannte Proteinkomplexe können durch Isolierung und Mikrosequenzierung analysiert werden. Dieses Verfahren weist eine Reihe von Limitationen und Nachteilen auf, da für die Untersuchungen große Mengen von Erregermaterial notwendig sind. Eine Analyse direkt aus der Primärläsion ist nicht möglich, sondern erst nach z.T. sehr langwieriger Kultur (z.B. bei Mykobakterien). Einige Erreger sind nicht einfach anzuzüchten, für unbekannte Erreger sind Kulturbedingungen oft nicht etabliert. Tote Erreger entziehen sich von vorneherein diesem Anreicherungsverfahren.

Ein weiterer Nachteil der 2-D-Gel-Technologie besteht darin, dass sich der Genexpressionstatus eines Erregers in Zellkultur deutlich von dem *in vivo* unterscheidet. Viele pathogene Genprodukte werden erst bei der Invasion des Erregers in den Wirtsorganismus eingeschaltet. Damit stehen für die Untersuchung lediglich solche Proteine zur Verfügung, die im Infektionserreger zum Zeitpunkt der Kultur exprimiert werden. Damit wird eine Reihe von Proteinen, die nur im Wirt unter Infektionsbedingungen in detektierbarer Menge exprimiert werden, von der Untersuchung ausgeschlossen. Gerade solche können jedoch für eine diagnostische Serologie, die eine klinisch nicht relevante Kolonialisierung von einer invasiven Infektion unterscheiden können muss, relevant sein.

Weiterhin werden mittels 2-D-Gelen Antigene als Proteine identifiziert. Die Nukleotidsequenz, die Grundlage für viele nachfolgende Analysen ist, muss erst noch ermittelt werden.

Als Alternative zum 2-D-Gel-Verfahren können bei Erregern, deren gesamte Nukleotidsequenz bekannt ist, alle putatitiven Gene in Expressionskassetten eingerührt, rekombinant exprimiert und auf Immunogenität oder Antigenität untersucht werden (Pizza et al. (2000), Science 287(5459):1816-20). Die rekombinant exprimierten Gene werden z.B. in einem parallelisierten Dot-Blot Verfahren mit Immunseren gescreent.

Die Nachteile dieses Verfahrens bestehen darin, dass lediglich Proteine, von denen bekannt ist, dass sie im interessierenden Erreger exprimiert werden, der Analyse zugänglich sind. Erreger mit unbekannter Nukleotidsequenz entziehen sich dieser Analyse.

Da die oben genannten Untersuchungstechnologien material-, zeit-, personal- und kostenaufwendig sind, sind sie nur wenigen großen Zentren vorbehalten.

Eine effiziente und potentiell wirksame Alternative zu den "Proteomics"-Ansätzen stellt das Immunoscreening genomischer Expressionsbanken (z.B. Pereboeva et al. (2000), J. Med. Virol. 60: 144-151) dar. Allerdings müssen auch hierfür Infektionserreger unter definierten Kulturbedingungen angereichert werden. Das Genom der Erreger wird anschließend isoliert, enzymatisch oder mechanisch in Fragmente geschnitten und in Expressionsvektoren kloniert. Die exprimierten Fragmente können dann mit Seren daraufhin überprüft werden, ob sie durch Seren von infizierten Organismen erkannt werden. Der Vorteil dieses Verfahrens ist, dass es kosteneffektiv und schnell zur Identifikation von Antigenen führen kann. Jedoch ist auch für dieses Verfahren aufgrund der großen Menge an benötigten Erregemukleinsäuren unumgänglich, dass die Erreger durch *in vitro* Kultur vermehrt und anschließend aufgereinigt werden. Damit ist das Verfahren bisher auf Erreger limitiert, für die Kultur- und Aufreinigungsmodalitäten bekannt und etabliert sind.

Ein Problem ist die Identifikation von bis dato nicht oder nur unzureichend charakterisierten Infektionserregern. Insbesondere beschäftigt sich die vorliegende Erfindung mit den folgenden Aufgabenstellungen:
1) Entzündliche Erkrankungen mit aufgrund von Epidemiologie und klinischem Verlauf als wahrscheinlich anzunehmender infektiöser Ursache, für die bisher mit bekannten Verfahren Erreger nicht definiert bzw. nur unzureichend charakterisiert werden können. Hierzu zählen Erkrankungen wie Multiple Sklerose, Kawasaki-Disease, Sarkoidose, Diabetes mellitus, Morbus Whipple, Pityriasis rosea u.a. Wünschenswert für diese Erkrankungen ist ein Verfahren, welches eine systematische Analyse auf bisher unbekannte Infektionserreger von primärem Patientenmaterial, z.B. Lymphknoten-Biopsien, erlaubt.
2) Newly emerging infectious diseases. Hierzu zählen Infektionskrankheiten, die durch bisher nicht bekannte (z.B. HIV in den 80er Jahren) oder nicht gut charakterisierte Krankheitserreger verursacht werden und aufgrund z.B. Änderung der Epidemiologie plötzlich im Focus des klinischen Interesses stehen. Medizinisch und sozioökomisch essentiell für diese Klasse von Infektionserkrankungen ist, dass schnell Erreger identifiziert und entsprechende Diagnostika und eventuell auch Impfstoffe produziert werden können. Da im allgemeinen die Etablierung der Kultivierungsbedingungen für nicht charakterisierte Erreger bis zu mehrere Jahre in Anspruch nehmen kann, ist auch in diesem Fall eine Identifikation von Erregern und Erregerantigenen direkt aus dem infizierten Gewebe sehr wünschenswert, kann aber durch bekannte Verfahren nicht geleistet werden.

Es besteht daher ein großer Bedarf an einem Verfahren, welches die direkte Verwendbarkeit von Primärmaterial zur Erregeridentifikation ohne Notwendigkeit einer Erregervermehrung durch Kultur erlaubt. Weiterhin sollte dieses Verfahren die effiziente Entdeckung bisher unbekannter Krankheitserreger in Primärmaterial ermöglichen.

### Zusammenfassende Beschreibung der Erfindung

Eine Aufgabe der vorliegenden Erfindung war es daher ein Verfahren zu entwickeln, welches eine Identifikation von Erregernukleinsäuren direkt aus einer limitierten Menge (z.B. 50 mm³) von infizierten Patientenmaterial ermöglicht.

Die vorliegende Erfindung beschreibt ein Verfahren zur systematischen Identifikation von bekannten wie auch unbekannten nukleinsäurekodierten Erregern und ihren Antigenen anhand der von ihnen ausgelösten Immunantwort im Wirtsorganismus.

Gegenstand der vorliegenden Erfindung ist daher ein Verfahren zur Identifizierung von biologisch aktiven Strukturen, die durch das Genom von mikrobiellen Erregern kodiert werden, ausgehend von genomischen Erregernukleinsäuren, wobei das Verfahren die folgenden Schritte umfasst: Gewinnung von genomischen Erregernukleinsäuren aus erregerhaltigen Proben, sequenzunabhängige Amplifikation von genomischen Erregernukleinsäuren mittels Polymerase-Kettenreaktion oder reverser Transkription, oder beiden und unter Verwendung von Oligonukleotid-Primern mit Zuffalsequenzen. Expression amplifizierter Erregernukleksäuren und Screening und Identifizierung der biologisch aktiven Struktur.

Das erfindungsgemäße Verfahren hat den entscheidenden Vorteil, dass auch bei sehr geringer Erregermenge eine umfassende Identifikation vom Wirtsorganismus erkannter Errergerantigene (mikrobielles Immunom) möglich ist.

Das erfindungsgemäße Verfahren ist dadurch gekennzeichnet, dass geringste Ausgangsmengen bis minimal 1 pg Erregernukleinsäure ausreichen, um eine effektive Analyse zu erzielen. In einer bevorzugten Ausführungsform werden 10-20 pg, insbesondere 1-10 pg an Erregernukleinsäure verwendet.

Die hohe Sensitivität des Verfahrens ermöglicht einerseits die Untersuchung von Erregern aus Primärisolaten ohne die Notwendigkeit, diese Erreger zuvor durch *in vitro* Kultur anreichern zu müssen. Somit können Erreger untersucht werden, die sich mit bekannten Methoden schwer (z.B. Mykobakterium tuberculosis U.G.) oder gar nicht anzüchten lassen (z.B. Mykobacterium leprae oder nicht-vitale Keime).

Andererseits wird durch den Wegfall der *in vitro* Anreicherung vermieden, dass es zu einer durch die *in vitro* Kultur bedingten Verfälschung der Keimpopulation kommt (z.B. durch Überwachsen relevanter pathogener Keime bei Mischinfektionen).

Weiterhin eröffnet die hohe Sensitivität des Verfahrens die Möglichkeit, ein breites Spektrum an Ausgangsmaterialien zur Erregerisolierung zu nutzen. Der Begriff "Proben" bezeichnet dabei verschiedene biologische Materialien wie Zellen, Gewebe, Körperflüssigkeiten. In einer bevorzugten Ausführungsform der Erfindung stellen Blut, Gewebe, kultivierte Zellen, Serum, Sekret aus Läsionen (Pusteln, Krusten etc.) und andere Körperflüssigkeiten wie z.B. Urin, Speichel, Liquor, Gelenkflüssigkeiten, Galle und Augendrüsensekrete bevorzugt verwendete Proben dar.

### Detaillierte Beschreibung der Erfindung

### Gewinnung von genomischen Erregernukleinsäuren aus erregerbaltigen Proben

Im ersten Schritt des erfindungsgemäßen Verfahrens werden genomische Erregernukleinsäuren aus erregerhaltigen Proben gewonnen.

Der hier verwendete Begriff "mikrobieller Erreger" umfasst virale und bakterielle Erreger. Erreger liegen in Wirtszellen oder im Zellverband mit Wirtszellen vor und müssen mit Ausnahme von frei im Serum zirkulierenden Erregern zugänglich gemacht werden. In einer bevorzugten Ausführungsform der Erfindung liegt der Erreger intrazellulär oder extrazellulär vor.

Intrazelluläre Erreger können durch Zellyse (z.B. mechanisch oder über Detergenzien, bei eukaryontischen Zellmembranen z.B. mittels SDS) freigesetzt werden. Bevorzugte SDS-Konzentration sind für grampositive Bakterien >0,05% bis 1% und für gramnegative Bakterien 0,05% bis 0,1%. Der Fachmann kann die geeignete Konzentration für andere Detergenzien leicht bestimmen, indem die Konzentration eingesetzt wird, bei der die Hülle, z.B die Bakterienwand, des grampositiven oder des gramnegativen Bakteriums noch intakt ist, die eukaryontische Zellwand aber bereits aufgelöst wird.

Extrazelluläre Erreger können z.B. über ihre deutlich geringere Partikelgröße (20 nm-1 µM) von Wirtszellen getrennt werden (z.B. durch Sedimentation/Zentrifugation und/oder Filtration).

In einer besonders bevorzugten Ausführungsform der Erfindung ist der Erreger nicht-infektiös und/oder nicht-vital. Die hohe Sensitivität des Verfahrens ermöglicht es, dass auch auf nichtinfektiöse und/oder nach Ablauf einer floriden Infektion auf residual verbliebene nicht-vitale Erreger zurückgegriffen werden kann.

Im folgenden wird der erste Schritt des erfindungsgemäßen Verfahrens, die Gewinnung von genomischen Erregernukleinsäuren aus erregerhaltigen Proben, weiter beschrieben.

Die Gewinnung genomischer Erregernukleinsäuren aus erregerhaltigen Proben ist in einer bevorzugten Ausführungsform der Erfindung durch die folgenden Schritte gekennzeichnet: Freisetzung von Erregerpartikeln aus erregerhaltigen Proben, nachfolgend Elimination und/oder Reduktion kontaminierender Wirtsnukleinsäuren und abschließend Extraktion der genomischen Erregernukleinsaure aus freigesetzten Erregerpartikeln.

Gemäss einer Ausführungsform der Erfindung erfolgt die Freisetzung von Erregerpartikeln durch Zellyse, Sedimentation, Zentrifugation und/oder Filtration.

Gemäss einer bevorzugten Ausführungsform erfolgt die Ellimination der kontaminierenden Wirtsnukleinsäuren dadurch, dass vor der Extraktion der Erregernukleinsäure ein RNase-und/oder DNase-Verdau durchgeführt wird.

Dies ist besonders dann vorteilhaft, wenn der Erreger ein durch Kapsidproteine geschütztes Virus ist. Da die Kapsidhülle von Viren einen Schutz vor Nukleasen bietet, sind die Erreger entsprechend vor der Aktivität extrazellulärer RNasen und DNasen geschützt.

Eine weitere Ausführungsform der Erfindung umfasst daher den Schritt der Elimination und/oder Reduktion kontaminierender Wirtsnukleinsäuren durch RNase und/oder DNase-Verdau besonders für virale Erreger (s. Beispiel Vacciniavirus). Die Dnase Behandlung zur Aufreinigung von Viruspartikeln ist dem Fachmann bekannt und kann durchgeführt werden wie beispielsweise beschrieben bei Dahl R, Kates JR. Virology 1970;42(2):453-62, Gutteridge WE, Cover B., Trans R Soc Trop Med Hyg 1973;67(2):254, Keel JA, Finnerty WR, Feeley JC., Ann Intern Med 1979 Apr;90(4):652-5 oder Rotten S. in Methods in Mycoplasmology, Vol. 1, Academic Press, 1983.

Eine weitere Möglichkeit, Erreger wie grampositive oder gramnegative Bakterien aus Geweben anzureichern, ist wie im erfindungsgemäßen Verfahren angewandt, eine differenzielle Lyse (nachfolgend auch als sequentielle Lyse bezeichnet) infizierter Gewebe mit Detergenzien wie z.B. SDS, die Lipidmembranen auflösen. Hierbei macht man sich den Umstand zu Nutze, dass die Zellmembranen eukaryontischer Wirtszellen sehr viel empfindlicher auf niedrige Konzentration von SDS reagieren und aufgelöst werden, während Bakterienwände widerstandsfähiger sind und ihre korpuskuläre Integrität erhalten bleibt.

Nach Anreicherung werden die Erregernukleinsäuren von den korpuskulären Bestandteilen des Erregers abgetrennt und aus dem Erreger freigesetzt. Dies kann mittels dem Fachmann bekannten Standardtechniken durchgerührt werden, wobei in einer bevorzugten Ausführungsform der Erfindung die Abtrennung durch Proteinase K-Verdau, Denaturierung, Hitze- und/oder Ultraschallbehandlung, enzymatisch mit Lysozym-Behandlung oder organische Extraktion erfolgt.

Die Aufreinigung von Bakterien/Viren durch Detergentien wie SDS kann durchgeführt werden wie bei Takumi K, Kinouchi T, Kawata T., Microbiol Immunol 1980;24(6):469-77, Kramer VC, Calabrese DM, Nickerson KW., Environ Microbiol 1980 40(5):973-6, Rudoi NM, Zelenskaia AB, Erkovskaia., Lab Delo 1975;(8):487-9 oder Keel JA, Finnerty WR, Feeley JC., Ann Intern Med 1979 Apr;90(4):652-5, s.a. US Patent 4,283,490.

Die Erfindung löst das Problem, dass in einem infizierten Gewebe / Organ nur ein Teil des Gewebes/Zellen mit dem Erreger infiziert ist.

Die vorliegende Erfindung ist auch geeignet zum Nachweis von Erregern bei Fällen wie z.B. Mykobakteriosen, wo nur wenige bis einzelne Erregerpartikel in den infizierten Geweben existent sind.

Die vorliegende Erfindung hat den Vorteil, dass in den Fällen einer Infektion mit nur einer geringen Totalmenge an Erregernukleinsäuren pro Gewebseinheit (z.B. 50 mm³) der Nachweis der Erregers möglich ist. Als Zahlenbeispiel für DNA-kodierte Pathogene lässt sich errechnen, dass ca. 10⁶ Viren mit einer durchschnittlichen Genomgrösse von 10000 Basen bzw. 10⁴ Bakterien mit einer durchschnittlichen Genomgröße von 1.000.000 Basen gerade 10 pg Erregernukleinsäuren beinhalten. Zudem ist das Genom der meisten Infektionserreger deutlich kleiner als das menschliche Genom (bis Faktor 10⁶ bei Viren, bis Faktor 10⁴ bei Bakterien). Dadurch verdünnt sich der Anteil der Gesamtmenge an Erregernukleinsäuren gegenüber der Gesamtmenge der Wirtsnukleinsären je nach Kopienzahl und Genomgrösse des Erregers um eine vielfache Zehnerpotenz (Verhältnis Wirtsnukleinäure / Erregernukleinsäure 10⁴ bis 10⁹).

### Sequenzunabhängige Amplifikation von genomischen Erregernukleinsäuren

Das erfindungsgemäße Verfahren umfasst weiterhin die sequenzunabhängige Amplifikation genomischer Erregernukleinsäuren mittels Polymerase-Kettenreaktion (PCR), die der Vermehrung des Ausgangsmaterials dient.

Hierbei werden PCR-Primer mit Zufallssequenzen (Random-Oligonukleotide) verwendet, so dass nicht nur spezifische Genbereiche, sondern möglichst das gesamte Genom des Erregers repräsentativ amplifiziert werden kann (unselektive Nukleinsäureamplifikation mit degenerierten Oligonukleotiden). Diese Primer binden natürlich auch an Wirts-DNA Diese wurden jedoch, wie oben beschrieben, durch vorhergehenden RNnase- und/oder DNase-Verdau bzw. durch selektive Zelllyse reduziert bzw. eliminiert.

Allerdings ist Eliminierung bzw. Reduktion von Wirts-DNA durch vorhergehenden RNnase-und/oder DNase-Verdau bzw. durch selektive Zelllyse in dem erfindungsgemäßen Verfahren nicht zwingend erforderlich. Bei einer hohen Konzentration der Erreger- DNA oder RNA, wie zum Beispiel in Pusteln oder Virusbläschen, ist eine Eliminierung bzw. Reduktion von Wirts-DNA nicht erforderlich.

Der hier verwendete Begriff "genomische Erregernukleinsäure" umfasst sowohl genomische DNA als auch RNA.

In einer bevorzugten Ausführungsform der Erfindung ist die genomische Erregernukleinsäure DNA, und ihre sequenzunabhängige Amplifikation erfolgt durch Klenowreaktion mit Adaptoroligonukleotiden mit degeneriertem 3'-Ende und nachfolgender PCR mit Oligonukleotiden, die der Adaptorsequenz entsprechen.

In einer weiteren bevorzugten Ausführungsform ist die genomische Erregernukleinsäure RNA, und ihre Amplifikation erfolgt durch reverse Transkription mit degenerierten Oligonukleotiden und nachfolgende Amplifikation durch PCR.

Wenn nicht bekannt ist, ob der Erreger durch RNA oder DNA kodiert wird, werden beide Reaktionen in getrennten Reaktionsgefäßen angesetzt und getrennt amplifiziert. Die Amplikons repräsentieren in beiden Fällen genomische Nukleinsäurefragmente. Durch die Verwendung von degenerierten Oligonukleotiden wird eine zufällige Amplifikation (Shotguntechnik) des Gesamtgenoms ermöglicht.

Die Stärke des erfindungsgemäßen Verfahrens liegt in seiner hohen Sensitivität und Effizienz (Ausgangsmengen von wenigen picogramm reichen aus) bei gleichzeitig guter Erhaltung der Repräsentanz sämtlicher Bereiche des Gesamtgenoms. Die gute Repräsentanz der mikrobiellen Genabschnitte in den nach dem erfindungsgemäßen Verfahren generierten Bibliotheken wird durch Variationen in der Zwei-Schritt-PCR, wie z.B. Veränderungen der Salzkonzentration, erreicht. Die hohe Effizienz der Methode mit weitgehender Erhaltung der Repräsentanz erübrigt daher das Anlegen einer Primärkultur zur Vermehrung des Erregers mit allen durch sie bedingten Limitationen. Im Falle von unbekannten Erregern sind Kulturbedingungen nicht definiert und müssten im trial-and-error-Verfahren approximiert werden. Auch ist eine Reihe bekannter Erreger schwierig anzuzüchten. Bei Mischinfektionen können Primärkulturen durch Überwachsungsphänomene gerade die relevante Erregerpopulation ausdünnen. Tote Erreger würden gar nicht erfasst. Diese Nachteile anderer Techniken werden durch das erfindungsgemäße Verfahren umgangen.

In einer bevorzugten Ausführungsform wird die Sequenz-unspezifische Amplifikation von Erregernukleinsäuren in zwei sequentiell hintereinandergeschalteten PCR-Schritten von jeweils 35-40 Zyklen durchgeführt.

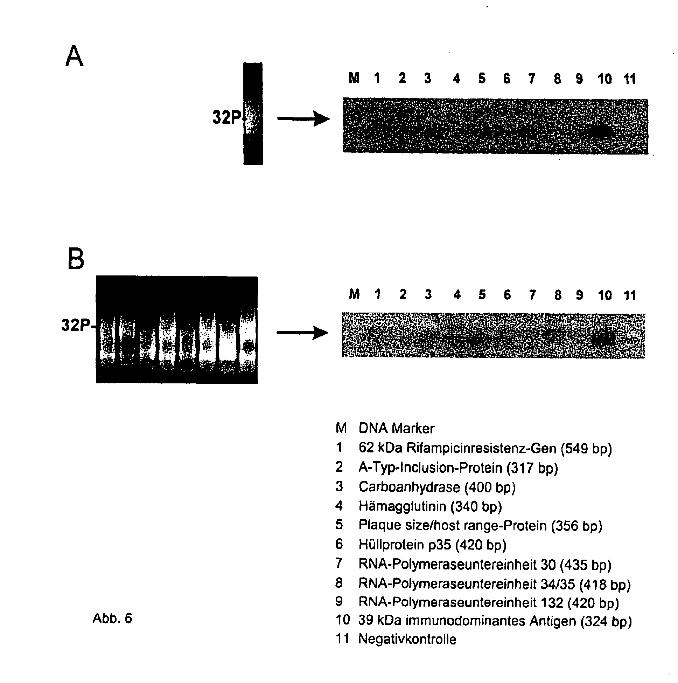

Hierzu wird nach der ersten Amplifikation 1/20 bis 1/50 des Volumens der ersten PCR für die Reamplifikation unter variierenden Bedingungen (z.B. Variation der MgCl Konzentration, der Pufferbedingungen, der Poylmerasen) genutzt. Durch die Reamplifikation in einer zweiten PCR wird wie im Beispiel 3A dargestellt, eine höhere Sensitivität des Verfahrens gewährleistet. Die Variation der Re-Amplifikationsbedingungen ermöglicht (siehe Beispiel 6, Abbildung 6) eine besonders gute Repräsentation unterschiedlicher Abschnitte des Erregergenoms und damit eine umfassende Analyse des Erregers.

### Expression amplifizierter Erregernukleinsären

Auf die Amplifikation der genomischen Erregernukleinsäuren folgt deren Expression. Dazu werden die Erregernukleinsäuren zur Erstellung einer genomischen Expressionsbank des Erregers in geeignete Expressionsvektoren kloniert.

In einer Ausführungsform der Erfindung sind die Expressionsvektoren ausgewählt aus der Gruppe der viralen, eukaryotischen oder prokaryotischen Vektoren. Im Rahmen der Erfindung können alle Systeme genutzt werden, die eine Expression von rekombinanten Proteinen erlauben.

Nach dem Einbringen der Erregennukleinsäuren in die Vektoren werden die Vektoren bevorzugt in Lambda-Phagen verpackt.

In einer bevorzugten Ausführungform der Erfindung wird die Expression der Erregernukleinsäuren durch Einbringen der Erregernukleinsäure in Lambda-Phagen Vektoren (z.B. Expressionsvektor Lambda-ZAP-Express, US Patent Nr. 5,128,256) gewährleistet. Alternativ können auch andere Vektoren, die dem Fachmann bekannt sind, eingesetzt werden, besonders bevorzugt sind filamentöse Phagen-Vektoren, eukaryontische Vektoren, retrovirale Vektoren, adenovirale Vektoren oder Alphavirusvektoren.

### Screening und Identifizierung der biologisch aktiven Struktur

Der abschließende Schritt des erfindungsgemäßen Verfahrens umfasst das Screening der genomischen Expressionsbank und die Identifizierung der biologisch aktiven Struktur des Erregers durch Immunantworten infizierter Wirte.

Der hier verwendete Begriff "biologisch aktive Struktur" bezeichnet Erregerantigene, enzymatisch aktive Proteine oder Pathogenitätsfaktoren des mikrobiellen Erregers.

Gemäß einer bevorzugten Ausführungsform der vorliegenden Erfindung stellt das Screening ein Immunoscreening auf Erregerantigene dar und die Identifikation von Erregerantigenen umfasst die folgenden Schritte: Infektion von Bakterien mit Lambda-Phagen, Kultivierung der infizierten Bakterien unter Bildung von Phagenplaques, Transfer der Phagenplaques auf eine Nitrozellulosemembran (oder eine andere Festphase, die zur Immobilisierung von rekombinanten, aus den Erregern abgeleiteten Proteinen geeignet ist), Inkubation der Membran mit Serum oder antikörperhaltigen Körperflüssigkeiten des infizierten Wirtes, Waschen der Membran, Inkubation der Membran mit sekundärem alkalische Phosphatasegekoppeltem anti-IgG-Antikörper, der spezifisch für Immunglobuline des infizierten Wirtes ist, Nachweis der mit Wirtsserum reaktiven Klone durch Farbreaktion, und Isolierung und Sequenzierung der reaktiven Klone.

Grundsätzlich ist es zur Identifikation der biologisch aktiven Struktur auch möglich, Erregernukleinsäuren in rekombinante filamentöse Phagen-Vektoren (z.B. pJufo) einzubringen, die eine Expression von Antigenen direkt auf den Oberflächen der filamentösen Phagen erlauben. In diesem Fall würde die Identifikation von Erregerantigenen die folgenden Schritte umfassen: Generierung von rekombinanten filamentösen Phagen durch Einbringen der filamentösen Phagenvektoren in Bakterien, Inkubation generierter rekombinanter filamentöser Phagen mit Serum eines infizierten Wirtes, Selektion der filamentösen Phagen, an die Immunglobuline des Wirts gebunden haben, über immobilisierte Reagenzien, die spezifisch für die Immunglobuline des infizierten Wirts sind, und Isolierung und Sequenzierung der selektierten Klone.

Vom Erregergenom abgeleitete und rekombinant exprimierte Proteine können z.B. auf Festphase gebunden oder im Rahmen eines Panning-/Captureverfahrens mit spezifischen Immunantwortäquivalenten des infizierten Wirtes gescreent werden. Dies sind zum einen Antikörper verschiedener Immunglobulinklassen/-subklassen, in erster Linie IgG. Zu diesem Zweck wird Wirtsserum verwendet. Dies sind aber auch spezifische T-Lymphozyten gegen MHC-restringiert erkannte Epitope von Erregerantigenen, die in einem eukaryotischen System getestet werden müssen.

Die Herstellungsbedingungen für die genomische Bank erfolgt so, dass die inserierten Fragmente aufgrund der Eigenart der PCR-Primer nach dem Zufallsgeneratorprinzip entstehen. Entsprechend sind Bereiche aus bekannten Antigenen repräsentiert, die natürlicherweise auch als Proteine gebildet würden. Auch Fragmente aus normalerweise nicht exprimierten intergenen Bereichen können entstehen, die je nach Länge offener Leseraster zur Expression kurzer Nonsensproteine oder -peptide führen können. Ein wichtiger Aspekt ist, dass im erfindungsgemäßen Verfahren automatisch auch bisher nicht identifizierte Erregerproteine vorliegen können.

### Detalierte Beschreibung bevorzugter Ausführungsformen der vorliegenden Erfindung

Die vorliegende Erfindung kombiniert die Expression der Gesamtdiversität aller erdenklicher rekombinanter Proteine mit der anschließenden Anwendung eines sehr stringenten Filters, nämlich die in infizierten Wirten im Rahmen des natürlichen Verlaufes der Erkrankung entstehende spezifische Immunantwort.

In einer Ausführungsform der Erfindung werden die Erreger vor der Nukleinsäureamplifikation durch Fällung mit Polyethylenglykol, Ultrazentrifugation, Gradientenzentrifugation oder durch Affinitätschromatographie angereichert. Dieser Schritt ist jedoch nicht zwingend vorgesehen.

Die Fällung mit Polyethylenglykol ist vor allem bei viralen Partikeln effizient. Alternativ bietet sich bei bekannten Erregern die Affinitätschromatographie unter Nutzung von erregerspezifischen Antikörpern gegen definierte und stabile Oberflächenstrukturen an. Eine weitere Alternative ist bei unbekannten Erregern die Nutzung des polyklonalen Patientenserums selbst möglich, wobei das polyklonalen Patientenserums auf der Festphase immobilisiert und zur Affinitätsanreicherung von Erregern als spezifisches Capture-Reagenz verwendet wird.

Das hier beschriebene Verfahren kann als Plattformtechnologie eingesetzt werden, um hocheffizient Antigen-kodierende Erregernukleinsäuren aus geringsten Mengen erregerhaltigen Materials zu identifizieren. Wie in den nachfolgenden Beispielen gezeigt, sind 1 bis 20 pg genomische Nukleinsäure ausreichend, um eine umfassende Identifikation des durch natürliche Immunantworten serologisch erkannten Antigenrepertoires einzelner Erreger zu leisten. So konnten mit der hier beschriebenen Technologie z.B. für das Vacciniavirus als immunodominant bekannte Antigene ausgehend von 20 pg DNA identifiziert werden.

Die geringe Menge an Nukleinsäuren, die für dieses Verfahren benötigt wird, ermöglicht die Anwendung des Verfahrens für medizinisch wichtige Fragestellungen, die mit den bisher bekannten Verfahren schlecht oder gar nicht bearbeitet werden konnten.

Hierzu zählt z.B. die systematische direkte Identifikation von Erregernukleinsäuren aus infizierten Zellen, z.B. empfängliche *in vitro* Zellinien, Organe, entzündliche Läsionen wie z.B. Pusteln auf der Haut, auf Schleimhäuten, aus infizierten inneren lymphatischen und nicht-lymphatischen Organen oder aus erregerhaltigen Flüssigkeiten (z.B. Speichel, Sputum, Blut, Urin, Eiter, Ergüsse), die von infizierten Organismen gewonnen werden. Ausgehend von einer Sensitivität von 1 pg sind je nach Genomgrösse des Erregers (z.B. Viren 3.000 - 250.000 bp oder Bakterien 100.000 - 5.000.000 bp) 50 bis 10⁵ Erregerpartikel ausreichend, um für Antigene kodierende Erregernukleinsäuren zu identifizieren. Es ist zu erwarten, dass in den meisten Fällen die Anzahl von Erregerpartikeln weit über der Sensitivitätsgrenze des erfindungsgemäßen Verfahrens liegt.

Die hohe Sensitivität des erfindungsgemäßen Verfahrens erlaubt, wie bereits vorstehend beschrieben, eine Untersuchung von Erregern aus Primärisolaten ohne Notwendigkeit einer *in vitro* Kultur. Besonders wichtig ist, dass sehr kleine Mengen von Ausgangsmaterial, wie z.B. Stecknadelkopf-grosse Biopsien oder wenige Microliter infizierte Proben-Flüssigkeiten für die erfolgreiche Anreicherung und Identifikation von biologisch aktiven Strukturen durch das erfindungsgemäße Verfahren ausreichen. Hiermit kann das erfindungsgemäße Verfahren auf jegliches Überschußmaterial aus der medizinisch-klinischen Diagnostik und auf kryoarchivierte Probenmaterialien zugreifen (im Beispiel 9 dargestellt).

Die Sequenzierung der anhand des erfindungsgemäßen Verfahrens identifizierten Erregernukleinsäure führt zur Identifikation des Erregers, aus dem die Nukleinsäure ursprünglich stammt. Damit ist eine vorherige Kenntnis des Erregers nicht notwendig und das Verfahren eignet sich zur Entdeckung von bisher nicht definierten Infektionserregern bzw. zu bisher nicht bekannten Antigenen.

Das Verfahren kann somit zur Untersuchung einer Reihe von Erkrankungen eingesetzt werden, bei denen ein Infektionserreger ätiologisch vermutet wird, aber noch nicht identifiziert werden konnte. Hierzu zählen Erkrankungen, welche die Koch'schen Postulate teilweise erfüllen (z.B. Übertragbarkeit), bei denen jedoch die Keime aufgrund von fehlender Anzüchtbarkeit/Isolierbarkeit der Erreger nicht identifiziert werden können. Weitere Beispiele sind Erkrankungen wie die Sarkoidose, Pitrysiasis rosea, Multiple Sklerose, Diabetis Mellitus und Morbus Crohn.

Auch bei einem Teil von Patienten mit ätiologisch unklarer chronischer Hepatitis (chronische non-B, non-C Hepatitis) wird eine bisher unbekannte Viruserkrankung vermutet. Die Keimzahlen im Serum von Patienten mit bekannter chronischer Virushepatitis sind hoch. So finden sich bei Patienten mit infektiöser chronischer HBV- bzw. HCV-induzierter Hepatitis in 1 ml Blut 10⁷-10⁸ Hepatitis B- bzw. Hepatitis C-Partikel. Unter der Annahme einer annähernd vergleichbaren Keimzahl ist das erfindungsgemäße Verfahren auch dazu geeignet, putative non-B, non-C Hepatitis-Erreger ausgehend aus geringen Mengen Blut/Serum (1-10 ml) infizierter Patienten zu identifizieren.

Der Schritt des Immunoscreening der vorliegenden Erfindung als hochsensitives und hochspezifisches Hochdurchsatz-Detektionsverfahren erlaubt die Identifikation einer Antigenkodierenden Nukleinsäure unter 10⁶-10⁷ nicht-immunogenen Klonen.

Hierzu reicht eine geringe Reinheit der Erregernukleinsäuren zur Identifizierung des Erregers aus. Diese geringe Reinheit kann problemlos durch verschiedene aus dem Stand der Technik bekannte Verfahren, wie z.B. Präzipitation von Erregerpartikeln mit Polyethylenglykol (PEG) und/oder Affinitätschromatographie und/oder Degradation von kontaminierenden Wirtsnukleinsäuren mit Nukleasen erreicht werden.

Eine zusätzliche Möglichkeit zur Anreicherung von Erregerpartikeln ist die Nutzung der in infizierten Organismen gegen Erregerpartikel gebildeten spezifischen Antikörper für Capture-Verfahren.

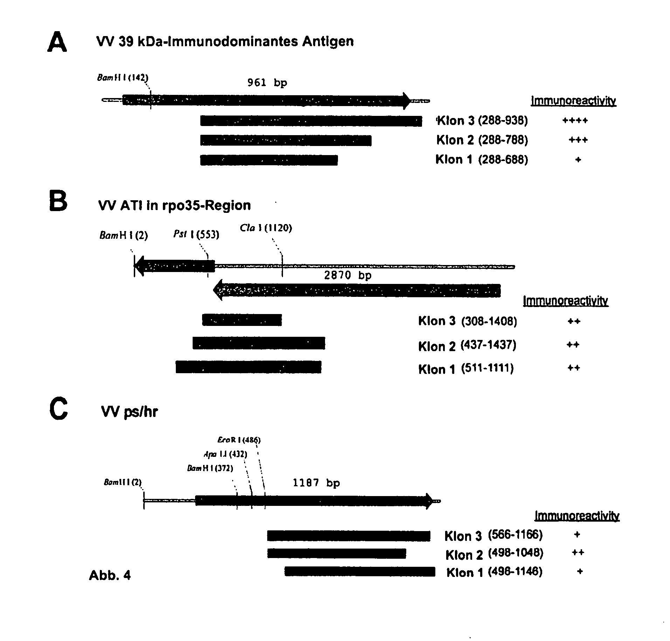

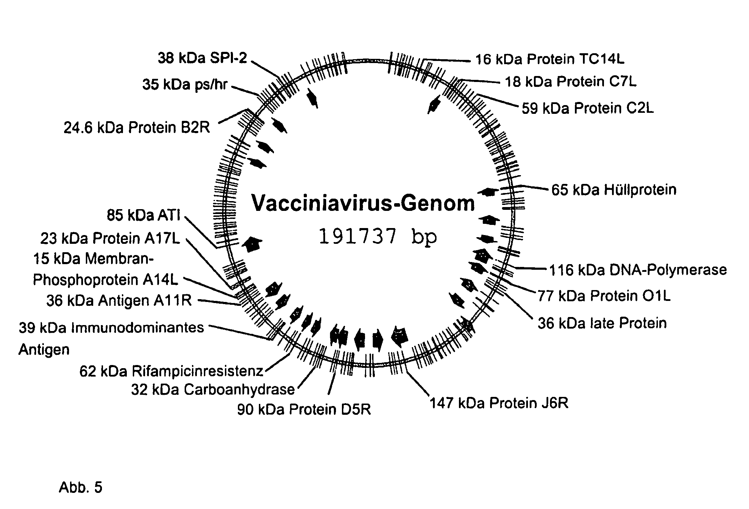

Das Immunoscreening als integraler Bestandteil des Verfahrens erlaubt die Analyse von 10⁶-5x10⁶ Klonen innerhalb von kurzer Zeit (2 Monaten) durch eine einzige Person. Die Kombination aus sequenzunabhängiger Amplifikation und serologischer Untersuchung mit hohem Durchsatz aller Nukleinsäureabschnitte in allen 6 Leserastern erlaubt schon bei mäßiger Reinheit der Ausgangsnukleinsäuren (Erregernukleinsäuren > 1% der Gesamtnukleinsäuren) die umfassende Untersuchung aller potentiell für Polypeptide kodierenden Regionen unabhängig vom aktuellen Expressionstatus. Durch die Untersuchung von genomischen Nukleinsäuren werden alle Genregionen erfasst, auch Gene, die nur zu bestimmten Zeitpunkten (z.B. nur in bestimmten Infektionszeitphasen) eingeschaltet werden. Dies erlaubt nicht nur eine Aussage über einzelne Antigene, sondern gibt auch Informationen über die Gesamtheit der Nukleotid-kodierten immunogenen Regionen (Immunom, siehe Abbildung 4A-C und 5). Über die Identifikation multipler z.T. überlappender Fragmente wird darüberhinaus eine Einengung des serologisch erkannten Epitops innerhalb eines identifizierten Antigens ermöglicht (siehe Abbildung 4A-C). Die Stärke des Signals ermöglicht eine weitere Diskriminierung von dominanten und nichtdominanten Epitopen (siehe Abbildung 4A-C). Die identifizierten Erreger-Nukleinsäurefragmente sind direkt für die Entwicklung von Serodiagnostika und Impfstoffen verfügbar. Die identifizierte Nukleinsäure kann als Matrize für die Entwicklung hochsensitiver direkter Erregemachweismethoden z.B. durch Anwendung von Nukleinsäure-spezifischer Amplifikation über die Polymerasekettenreaktion (PCR) genutzt werden. Die identifizierten Fragmente können auch für die Entwicklung von Diagnosetests basierend auf dem Nachweis von Antigen-spezifischen T-Lymphozytenreaktionen genutzt werden.

Die Abgrenzung gegen Technologien wie "Proteomics" ist schon in der Einleitung diskutiert worden. Das erfindungsgemäße Verfahren unterscheidet sich technisch gegenüber zwei weiteren verwandten Methoden: die serologische Untersuchung von genomischen Erregerbibliotheken und die SEREX-Technologie.

Für die serologische Untersuchung von genomischen Bibliotheken wurden von einigen Gruppen (z.B. Luchini et al. (1983), Curr Genet 10:245-52, Bannantine et al. (1998), Molecular Microbiology 28: 1017-1026) Expressionsbibliotheken aus aufgereinigter mechanisch zerkleinerter oder enzymatisch verdauter Erreger-DNA hergestellt. Für die Herstellung von Expressionsbibliotheken mit diesem Verfahren werden zur Herstellung repräsentativer Banken je nach Größe des Genoms zwischen 0,5 - 5 µg an aufgereinigten Erregernukleinsäuren benötigt (Faktor 10⁵-10⁶ Mehrbedarf an Erregemukleinsäuren). Hiermit verschließt sich die Methode der Untersuchung von Erregern aus Primärisolaten, in denen weitaus geringere Mengen an Erregern und Erregernukleinsäuren vorhanden sind. Es ist hier unumgänglich, dass die Erreger isoliert, *in vitro* angezüchtet und anschließend aufwendig aufgereinigt werden. Damit müssen als Grundvoraussetzungen zur Anwendung dieser Methode die Kultur- und Aufreinigungsmodalitäten für den jeweiligen Erreger bekannt und zuvor etabliert sein. Dies ist jedoch gerade für viele Viren und intrazelluläre Erreger technisch aufwendig und verlangt eine große Expertise. Damit entfällt auch die Möglichkeit, unbekannte Erreger, sowie solche, die nicht mehr vital sind, zu identifizieren.

Eine weitere Abgrenzung ist gegenüber der mit SEREX (Sahin et al. (1995), Proc Natl Acad Sci USA 92: 11810-3; Sahin et al. (1997), Curr Opin Immunol 9, 709-716) bezeichneten Methode vorzunehmen. Für SEREX wird aus erkrankten Geweben mRNA extrahiert, cDNA-Expressionsbibliotheken hergestellt und mit Seren desselben Individuums, aus dem das Gewebe stammt, auf immunreaktive Antigene gescreent.

Ein wesentlicher Unterschied zum erfindungsgemäßen Verfahren besteht darin, dass für die SEREX-Methode cDNA-Expressionsbibliotheken aus Wirtszellen infizierter Gewebe verwendet werden.

Die unterschiedliche Qualität und der unterschiedliche Ursprung der Nukleinsäuren führt zu folgenden Unterschieden:
Die Verwendung von Gesamt-mRNA aus Wirtszellen erhöht bei SEREX die Komplexität der Bibliothek und vermindert die Wahrscheinlichkeit der Identifikation Erreger-abgeleiteter Transkripte. Für tierische Wirtszellen ist von 40.000 bis 100.000 unterschiedlichen wirtseigenen Transkripten auszugehen. Die Anzahl der Transkripte für die meisten Erreger liegt weitaus geringer (Viren 3-200 Transkripte, Bakterien 500-4000 potentielle Genprodukte). Zusätzlich ist in den meisten Fällen nur ein geringer Teil von Wirtszellen mit Erregern infiziert, so dass der Anteil Erreger-abgeleiteter Nukleinsäuren in der Gesamt-mRNA Population sich weiter verdünnt. Da viele wirtseigene Transkripte auch für natürliche oder krankheitsassozierte Autoantigene kodieren (Sahin et al., 2000, Scanlan et al. (1998), Int J Cancer 76, 652-658) ist die Identifikation von Erreger-abgeleiteten Antigenen durch die präferentielle Detektion von Wirts-Gewebsautoantigenen sehr erschwert. Dieser Umstand ist verantwortlich dafür, dass mit der SEREX-Technologie bei der Untersuchung von mehreren infizierten Geweben, wie z.B. von HBV-Ag+ Leberzell-Karzinomen (Scanlan et al. (1998), Int J Cancer 76, 652-658; Stenner et al. (2000), Cancer Epidemiol Biomarkers Prev 9, 285-90) bisher noch keine Erregerantigene identifiziert worden sind.

Aus den nachfolgenden Abbildungen und Beispielen wird ersichtlich, dass mit dem Verfahren der vorliegenden Erfindung aus geringsten Mengen von Erregernukleinsäuren immunologisch relevante virale und bakterielle Antigene identifiziert und charakterisiert werden können. Die in den nachfolgenden Beispielen identifizierten viralen Antigene waren dabei über das gesamte Genom des Vacciniavirus verteilt, was auf eine gute Repräsentanz der verschiedenen Gene in der mit Hilfe des erfindungsgemäßen Verfahrens amplifizierten DNA schließen lässt (siehe Abb. 5). Eines der in den Beispielen identifizierten Antigene evoziert sogar neutralisierende Antikörper und ist somit im therapeutischen Zusammenhang von großer Wichtigkeit. Somit ist das Verfahren auch für die Auffindung von für die Therapie wichtiger Antigene geeignet.

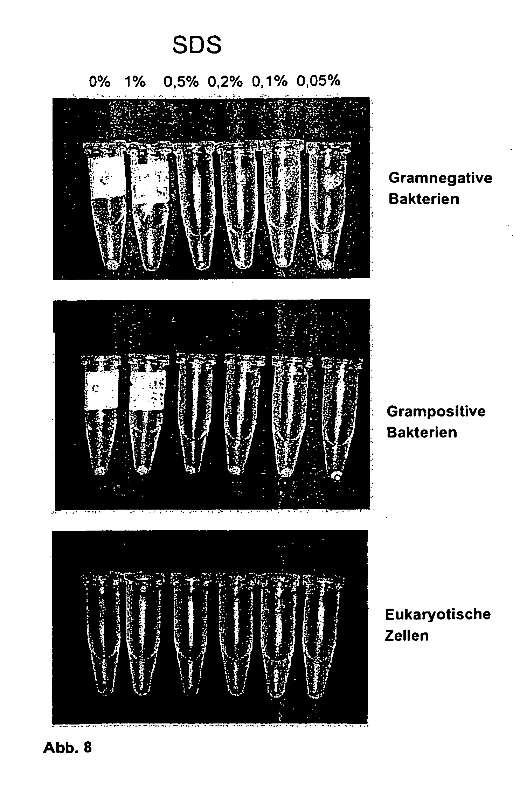

Das erfindungsgemäße Verfahren wurde in den vorliegenden Beispielen für die Identifikation von viralen und bakteriellen Antigenen eingesetzt. Erfindungsgemäß werden für bakterielle Erreger vorzugsweise die folgenden SDS-Konzentration zur Anreicherung von gramnegativen bzw. grampositiven Erregern eingesetzt (siehe Abb. 8): Für Gram-positive Bakterien >0,05% bis 1% und für Gram-negative Bakterien 0,05% bis 0,1% SDS.

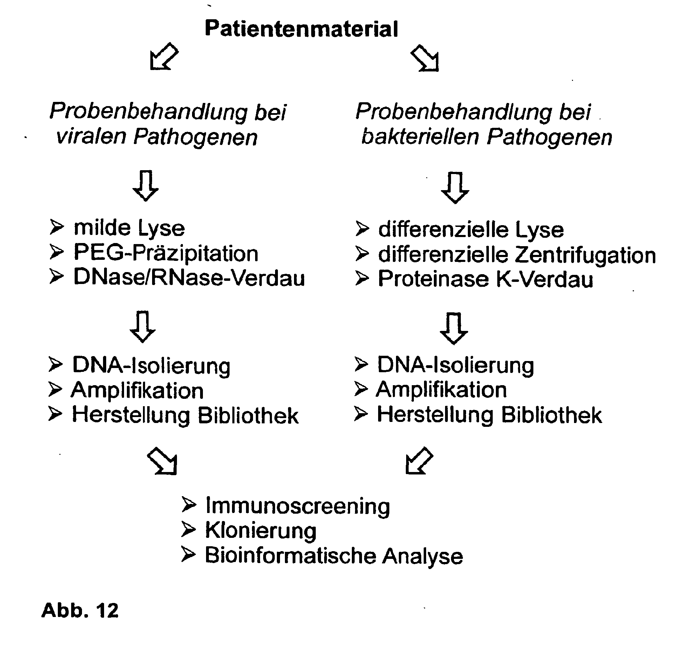

Wenn keine Anhaltspunkte vorliegen, ob ein Erreger ein Virus oder ein Bakterium ist, kann aufgrund der sehr geringen benötigten Materialmenge die Ausgangsprobe aufgeteilt werden (z.B. auf zwei Probengefäße) und unter der Annahme eines ursächlichen viralen bzw. bakteriellen Erregers methodisch unterschiedlich verarbeitet werden (Abb. 12).

Die folgenden Abbildungen dienen der Erläuterung der Erfindung:

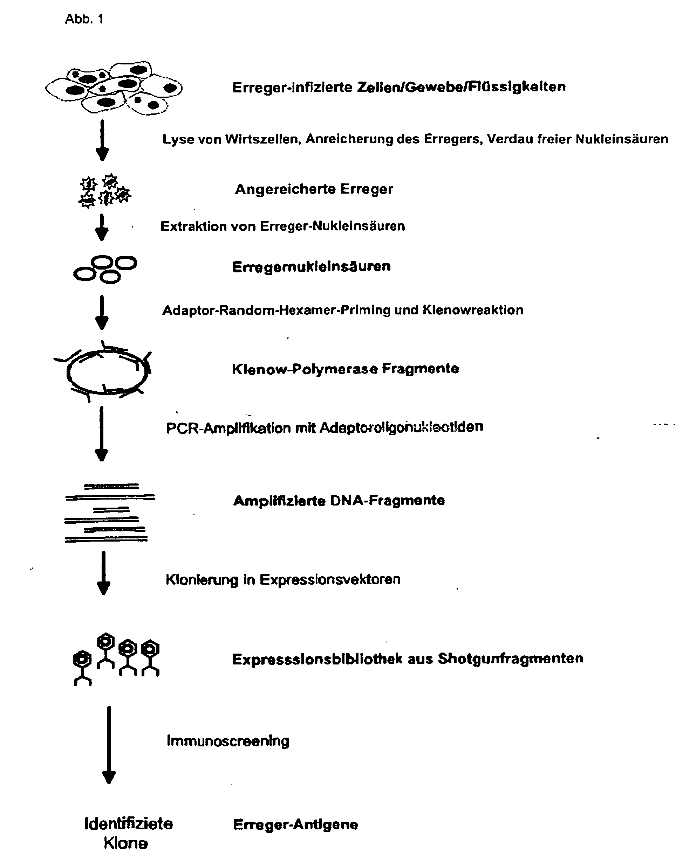

**Abb.1**. Schematische Darstellung der aufeinanderfolgenden Analyseschritte einer Ausführungsform des erfindungsgemäßen Verfahrens.

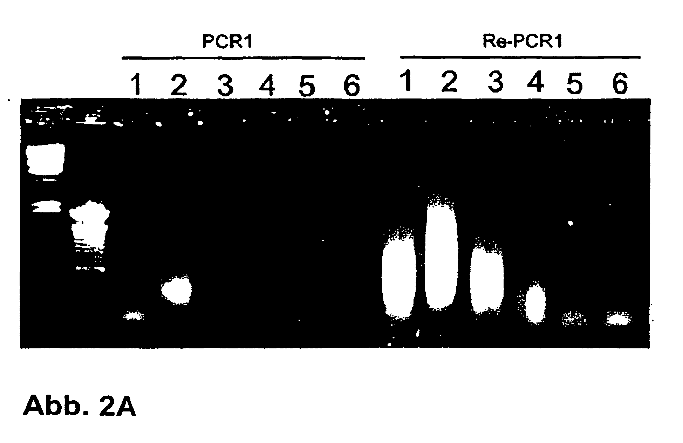

**Abb. 2A****. Amplifikation unterschiedlicher Ausgangsmengen von Erregernukleinsäuren.** Klenow-getaggte Vacciniavirus-DNA wurde wie im Beispiel 3A erläutert einmalig für 35 Zyklen amplifiziert (PCR1) bzw. 1 µl für weitere 35 Zyklen reamplifiziert (Re-PCR1). Als Ausgangsmengen für die Amplifikation dienten 1 ng (Spur 1), 40 pg (Spur 2), 8pg (Spur 3), 0,8pg (Spur 4), bzw 0,08pg (Spur 5) Klenow-Enzym getaggte Vacciniavirus-DNA Spur 6 stellt die Negativkontrolle ohne Zugabe von Vacciniavinus-DNA dar.

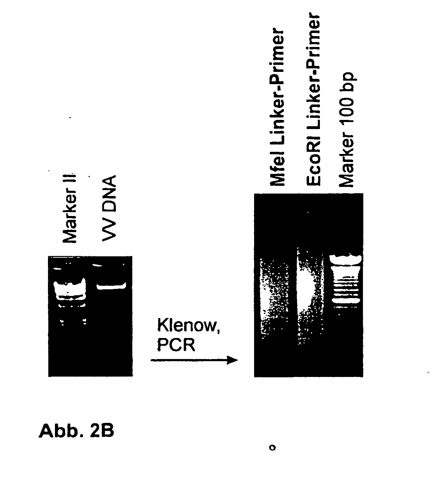

**Abb. 2B****.** Amplifikation von Vacciniavirus-DNA. Die Amplifikation der Vacciniavirus-DNA wurde durch eine Klenow-Enzym-Reaktion eingeleitet (links), für die Adaptoroligonukleotide mit degeneriertem 3'-Ende für ein sequenzunabhängiges Priming verwendet wurden. Darauf folgt die eigentliche PCR-Amplifikation mit Oligonukleotiden, die der Adaptorsequenz entsprechen. Die beschriebenen PCR-Bedingungen führten zu Fragmenten unterschiedlicher Länge (200-2500 bp) (rechts).

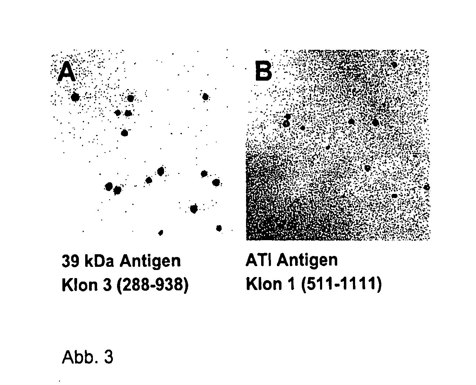

**Abb. 3****.** Immunoscreening und Identifizierung des 39kDa Antigens, Klon 3 (288-939) und des ATI Antigens Klon 1 (511-111). Anfänglich im Screening identifizierte Klone wurden zunächst oligoklonal unter Zunahme von benachbarten, nicht-reaktiven Phagenplaques isoliert und nach Bestätigung monoklonalisiert.
**Abb. 4A****.** Gezeigt sind Klon 1 (288-688), Klon 2 (288-788) und Klon 3 (288-938), die für überlappende Bereiche aus dem 39kDa-Protein von Vacciniavirus kodieren. Die Klone sind unterschiedlich immunreaktiv.
**Abb. 4B****.** Gezeigt sind drei Klone, die für überlappende Bereiche des A-Type-Inclusion-Proteins (ATI) von Vacciniavirus kodieren und gleiche Immunreaktivität aufweisen.
**Abb. 4C****.** Gezeigt sind drei Klone, die für überlappende Bereiche des Plaque size /host range - Proteins(ps/hr) von Vacciniavirus kodieren und unterschiedlich immunreaktiv sind.
**Abb. 5****.** Gezeigt ist die Verteilung der mit dem erfindungsgemäßen Verfahren identifizierten Klone im Vacciniavirusgenom. Die identifizierten Antigene sind über das ganze Vacciniagenom verteilt, was auf eine gute Repräsentanz der Vacciniavirusgene in der über das erfindungsgemäße Verfahren hergestellten Bibliothek schließen lässt.
**Abb. 6****.** Gezeigt ist die molekulare Analyse der Repräsentanz von zehn arbiträr ausgesuchten Vacciniavirusgenen in der durch das erfindungsgemäße Verfahren amplifizierten Vacciniavirus DNA. Zehn Genabschnitte aus dem Genom des Vacciniavirus wurden per PCR synthetisiert. Je 10 ng der 317 bis 549 bp langen Genabschnitte wurden durch Gelelektrophorese in Agarosegelen aufgetrennt und über das Southem Blotting-Verfahren auf eine Nylonmembran überführt, Für die Herstellung der ³²P-markierten Sonde wurden in 10-20 pg Vaciniavirus-DNA eingesetzt. Abb. 6A zeigt die Hybridisierung mit PCR-Fragmenten aus einer einzigen Re-PCR. Abb. 6B zeigt die Verbesserung der Repräsentanz, indem gepoolte Fragmente aus mehreren wie im Beispiel 2 beschrieben variierten Re-PCRs für die Hybridisierung eingesetzt wurden. Die Hybridisierung der gepoolten amplifizierten DNA mit den geblotteten zehn zufällig ausgewählten Abschnitten (sichtbar als schwache bis deutliche Schwärzung) des Vacciniavirusgenoms zeigt, dass alle Abschnitte in der amplifizierten DNA enthalten sind. Herauszustellen ist, dass selbst das Gen mit dem schwächsten Hybridisierungssignal (Spur 2, 94kDA A-Typ-Inclusion-Protein, ATI) im Immunoscreening der Bibliothek 30-mal als Antigen identifiziert wurde (s. Tabelle 3).

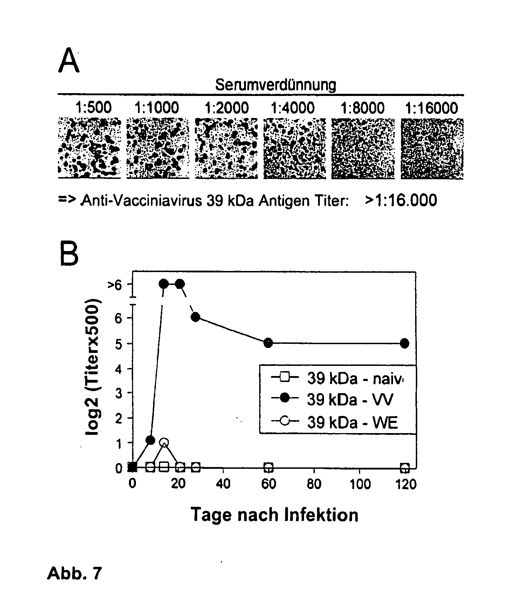

**Abb. 7A****.** Gezeigt ist die Bestimmung des Serumtiters gegen das durch das erfindungsgemäße Verfahren klonierte immundominante 39 kDa Antigen des Vacciniavirus. Serum von C57BL/6 Mäusen wurde am Tag 21 nach Infektion mit dem Vacciniavirus gewonnen und wie angegeben verdünnt. Zur Produktion des Antigens wurden E. coli Bakterien mit einem das 39 kDa Antigen kodierenden Lambaphagen infiziert. Die Reaktivität der Serumverdünnungen gegen das so rekombinant exprimierte 39 kDa Antigen wurde auf Nitrocellulosemembranen getestet. Für das hier verwendete Serum ist der Antikörpertiter >1:16000.
**Abb. 7B****.** Dergestellt ist der Verlauf des Antikörpertiters gegen das 39 kDa Antigen nach Infektion mit 2x10⁶ pfu Vacciniavirus oder mit 2x10⁵ pfu des Lymphocytären Choriomeningitisvirus (Stamm WE). Nicht infizierte Tiere (naiv) zeigen keine Reaktivität gegen das 39 kDa Antigen. Die hohe Spezifität der Reaktion ist auch durch die nur minimale Kreuzreaktivität mit dem Serum aus mit dem Lymphocytären Choriomeningitisvirus infizierten Mäusen (Tag 14) gezeigt.
**Abb. 8****.** Gezeigt ist die unterschiedliche Empfindlichkeit von eukaryontischen Zellen und gramnegativen bzw. grampositiven Bakterien. Für das Experiment wurden vergleichbare Zellvolumina von gramnegativen Bakterien (oben), grampositiven Bakterien (Mitte) bzw. eukaryontischen Zellen mit den angegebenen Konzentrationen von SDS inkubiert. Nicht lysierte korpuskuläre Strukturen wurden durch Zentrifugation pelletiert. Während eukaryontische Zellen bereits bei geringsten Konzentrationen vollständig von SDS lysiert werden (kein sichtbares Zellpellet, mikroskopisch keine sichtbaren Zellen) sind Bakterien widerstandsfähiger und können, da ihre korpuskuläre Integrität erhalten bleibt, durch Zentrifugation angereichert werden.

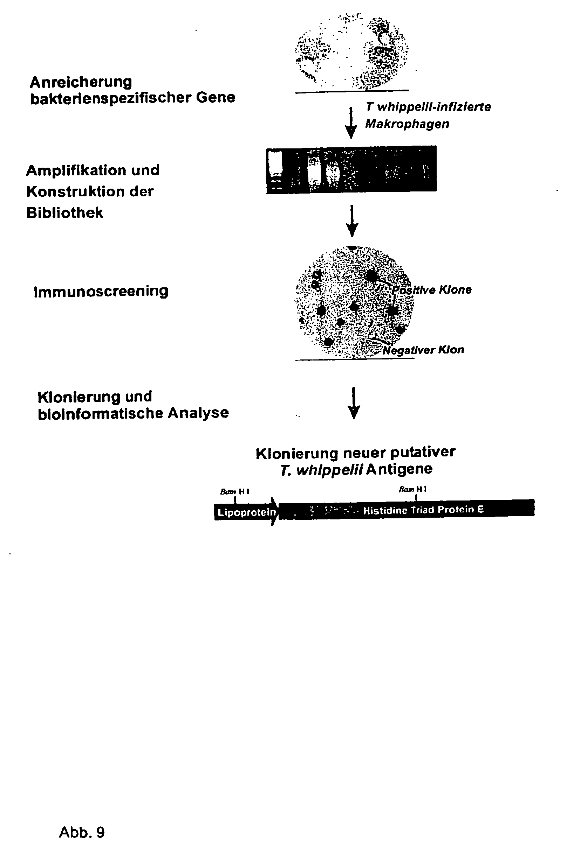

**Abb. 9****.** Gezeigt ist die Identifikation und molukulare Charakterisierung von putativen Antigenen des humanpathogenen Bakteriums Tropheryma whippelii. Interleukin-10 und Interleukin-4 deaktiverte humane Makrophagen wurde mit T. whippelii Bakterien enthaltendem Gehimmaterial eines an der Whippleschen Erkrankungen verstorbenen Patienten inkubiert. Bakterienspezifische Gene wurden durch differenzielle Lyse und anschliessende DNA-Aufarbeitung isoliert und Bibliotheken nach dem erfindungsgemäßen Verfahren konstruiert. Das Immunoscreening wurde mit Seren von T. whippelii infizierten Patienten durchgeführt. Die bioinformatische Analyse, d.h. der Abgleich mit öffentlich zugänglichen Sequenzdatenbanken, zeigt, dass bisher nicht bekannt bakterielle Antigene durch das erfindungsgemäße Verfahren identifiziert wurden.

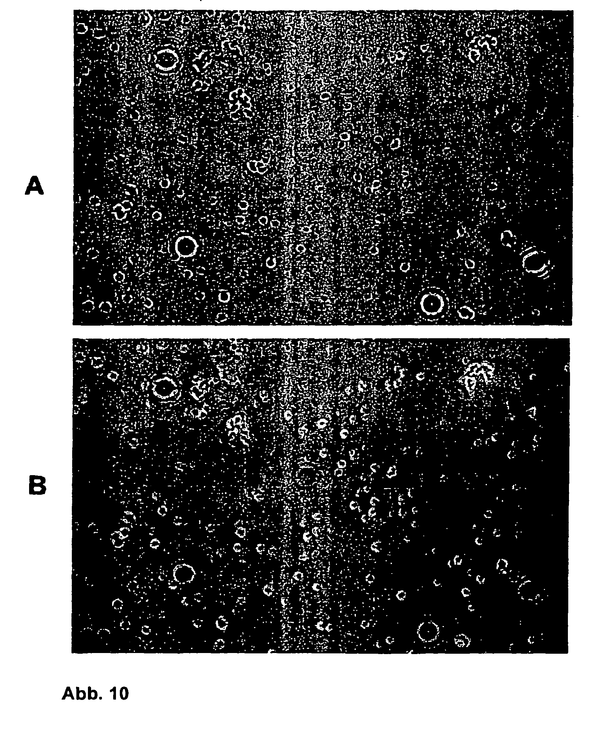

**Abb 10**: Isolation von Bakterien direkt aus dem Milz-Gewebe eines Patienten mit Whipple-krankheite. Bakterioen wurde aus einer kryopräservierten Milzprobe eines Patienten mit Whipple-krankheit wie im Beispiel 9 dargestellt isoliert und fluoreszensmikroskopisch analysiert. Das Bild zeigt die Überlagerung die Aufnahme im Phasenkontrast (Nachweis korpuskulärer Partikel, oben) und nach Überlagerung mit blauen Fluoreszenssignal (Nachweis DNA).

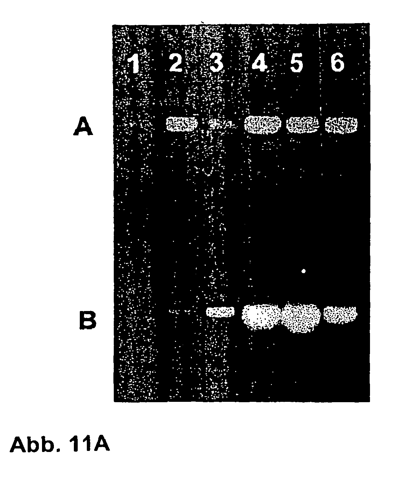

**Abb. 11A****:** Anreicherung von Erregernukleinsäuren direkt aus einer Patientenprobe wie in Beispiel 9 beschrieben.

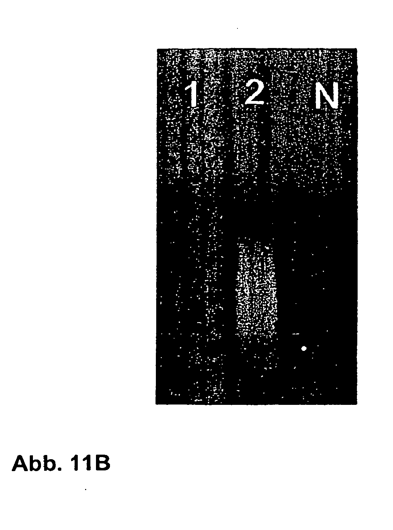

**Abb. 11B**: Amplifikation von direkt aus Patientenproben isolierten Erregernukleinsäuren. Die aus der Milzprobe angereicherte Bakterien-DNA wurde wie im Beispiel 10 beschrieben amplifiziert (Spur 2). Spur 1 stellt die Positivkontrolle mit einer anderen DNA-Probe dar. Auf Spur 3 ist die Negativkontrolle ohne Zusatz von DNA aufgetragen.
**Abb. 12****:** Schema eines möglichen Vorgehens für die Identifikation von Erregerantigenen für den Fall, dass nicht bekannt ist, ob der Erreger ein Virus oder ein Bakterium darstellt. Die Ausgangsprobe wird aufgeteilt und mittels unterschiedlicher, die Identifikation von bakteriellen bzw. viralen Erregern ermöglichenden Verfahren verarbeitet.

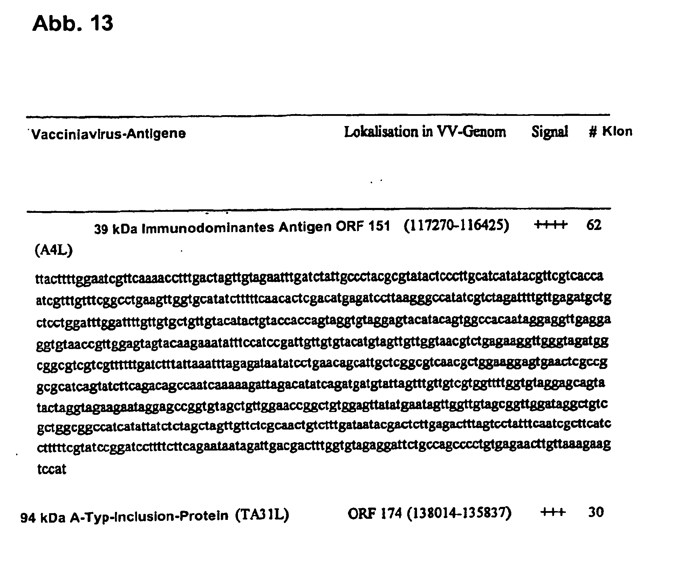

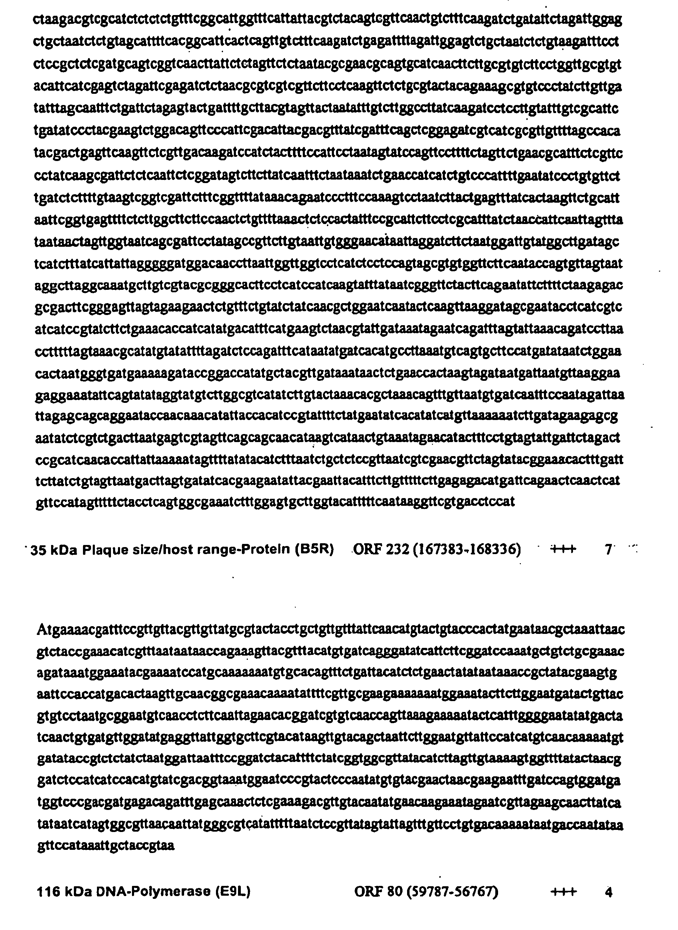

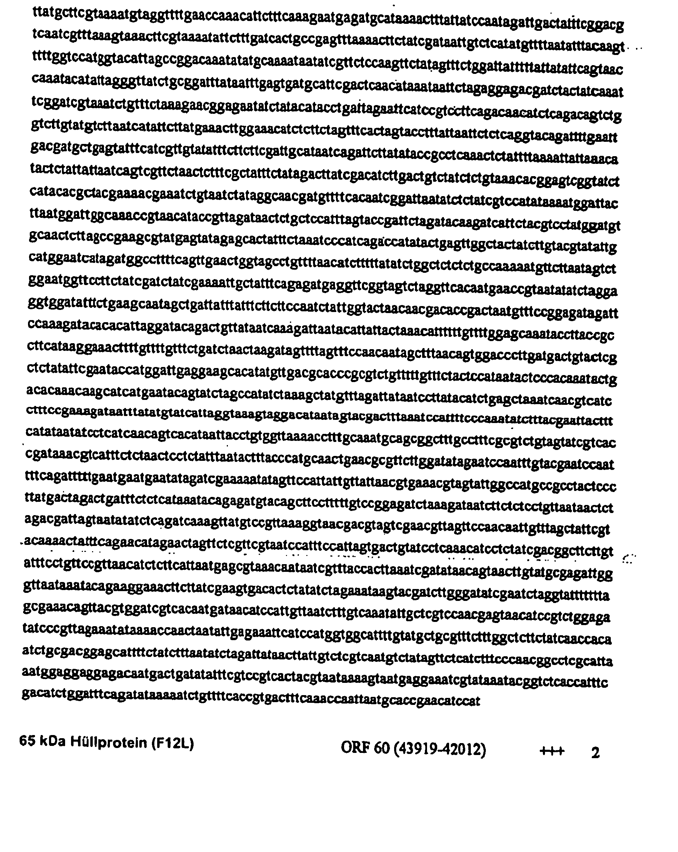

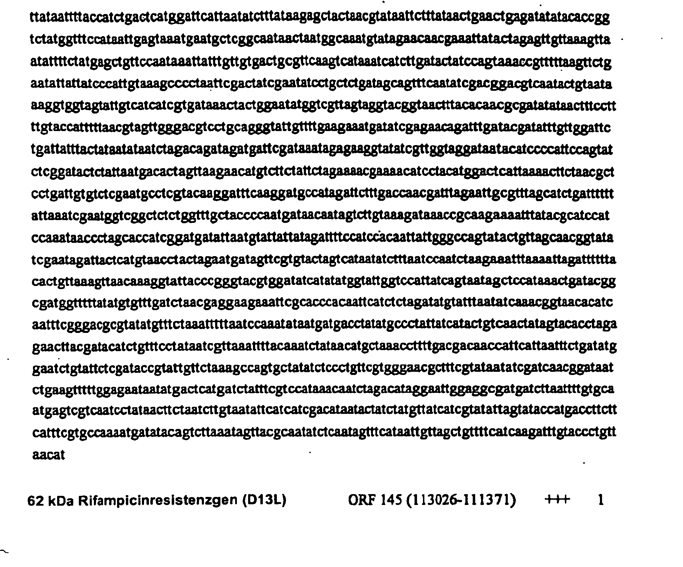

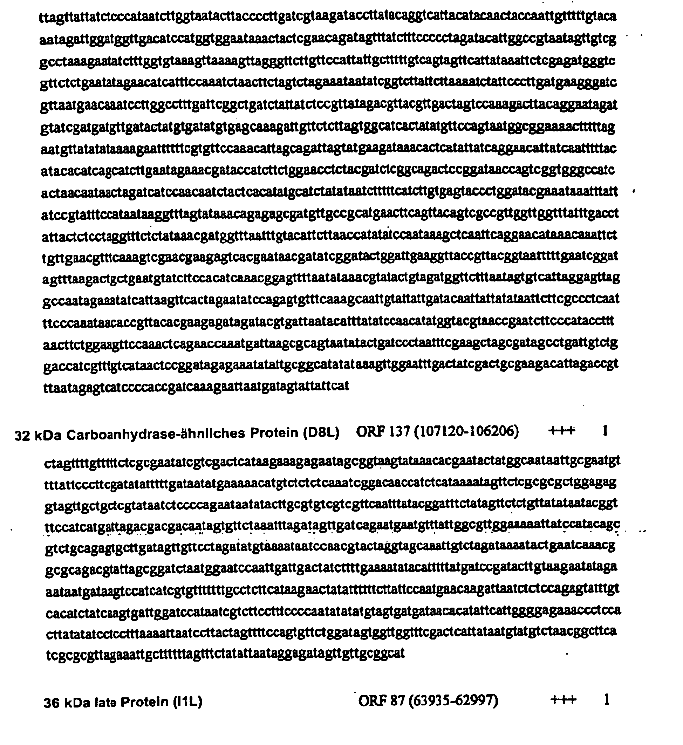

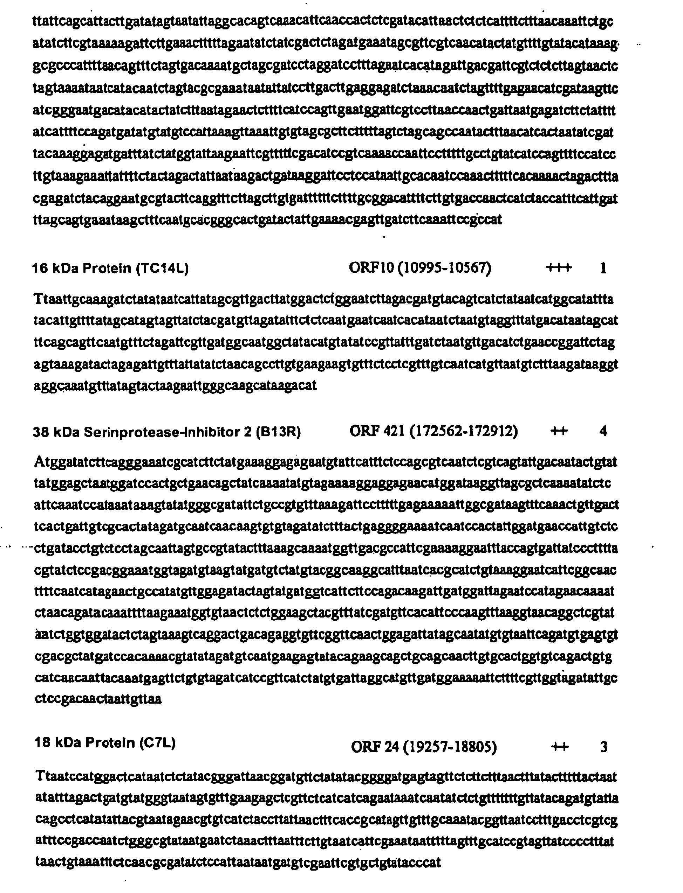

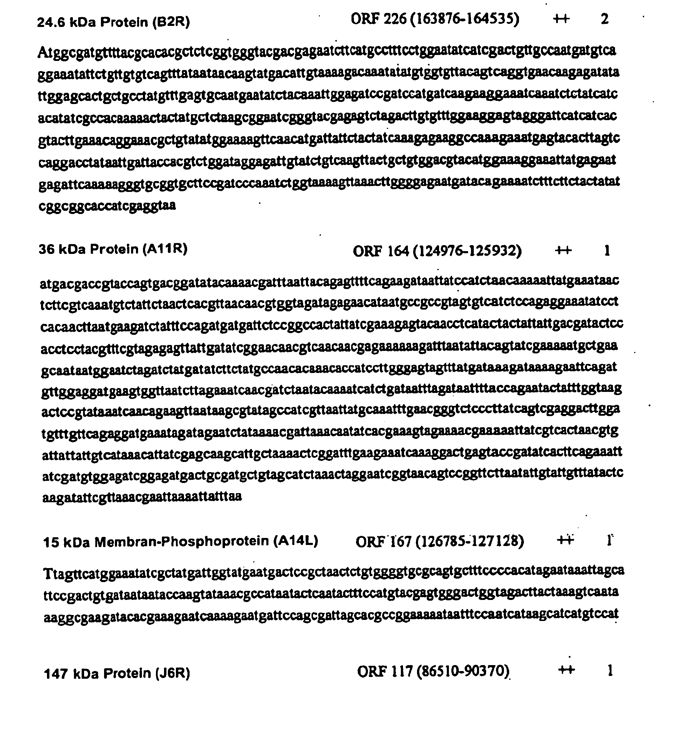

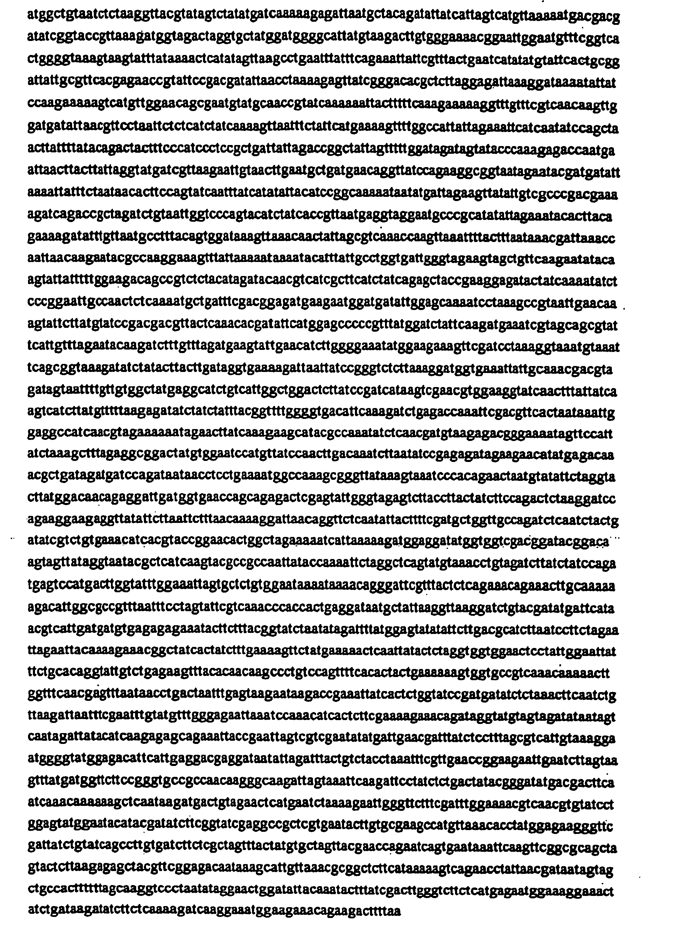

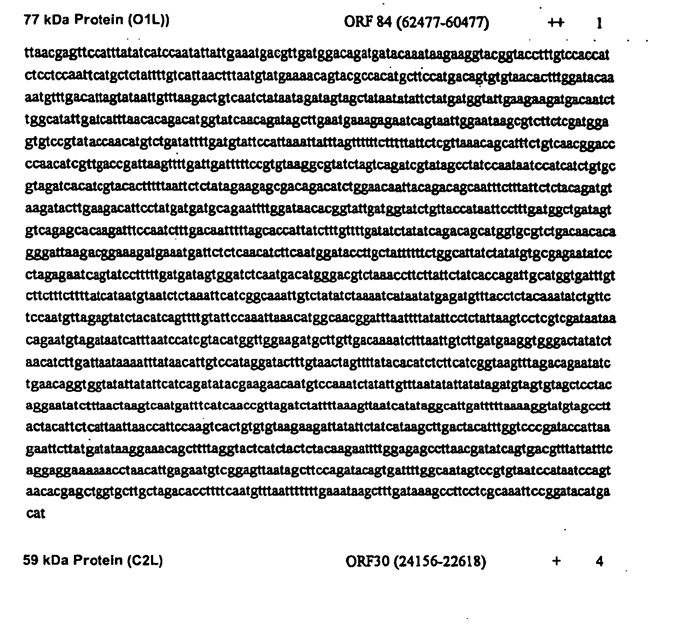

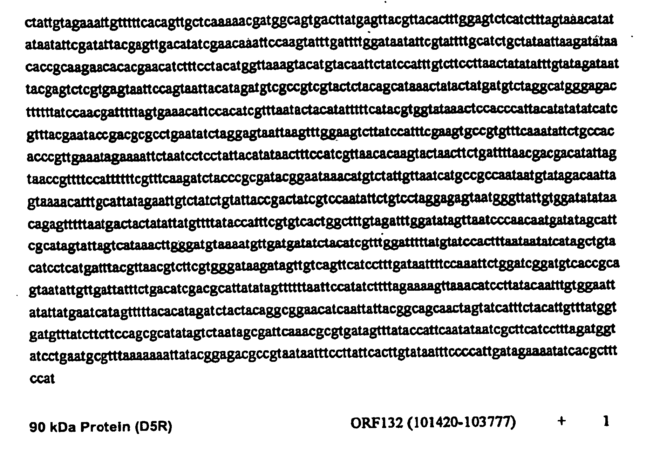

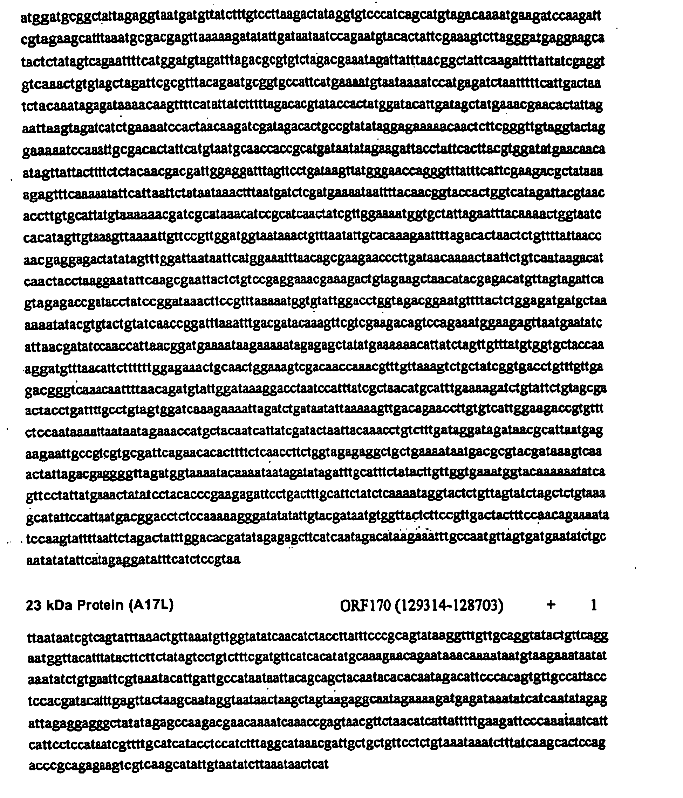

**Abb. 13**: Gezeigt sind die Nukleinsäuresequenzen der in Tabelle 3 aufgelisteten identifizierten Vacciniavirusantigene. Die Nukleinsäuresequenzen entsprechen den Sequenzen SEQ ID NO: 4 bis SEQ ID NO: 22 im Sequenzprotokoll.

Die vorliegende Erfindung wird weiterhin durch die folgenden Beispiele in nichtlimitierender Weise veranschaulicht.

### Beispiele

### Beispiel 1

### Isolierung von Erregernukleinsäuren aus Virus-infizierten Zellen

BSC40 Zellen wurden mit 2x10⁶ pfu Vacciniaviren infiziert. Infizierte Zellen wurden bei 37°C im CO₂-Inkubator 24 h inkubiert und geerntet. Geerntete Zellen wurden anschließend homogenisiert und in gepuffertem Medium aufgenommen. Zur Trennung von Viruspartikeln von Wirtszellfragmenten wurde das in Medium aufgenommene Zellysat anschließend mit Ultraschall behandelt. Grobpartikuläre Strukturen wurden durch Zentrifugation (15min x 3000 U/min) pelletiert. Nach Zentrifugation wurde der Überstand abgenommen und das Pellet verworfen. Um korpuskuläre Partikel im Überstand zu präzipitieren, wurde 2 ml gekühlter Überstand mit 6% PEG6000/0,6M NaCl präzipitiert (1h Inkubation auf Eis) und nachfolgend das Präzipitat mittels Zentrifugation bei 10000 x g für 10 min pelletiert. Da die vorhergehenden Schritte sowohl eine Abtrennung als auch eine Lyse kontaminierender Wirtszellen erbracht haben sollten, wurde jetzt nach Verwerfen des Überstandes das Präzipitat in 300 µl DNAse/RNAse Puffer aufgenommen und 30 min bei 37°C mit RNAse und DNAse verdaut. Hierdurch wurden die nun extrazellulär vorliegenden Nukleinsäuren der vorher desintegrierten Wirtszellen eliminiert. Die Virusnukleinsäuren werden durch die intakten Viruskapside vor den Nukleasen geschützt und nicht degradiert. Viruspartikel wurden durch vortexen mit 1 Volumen GITC-Puffer aufgeschlossen, was auch zur Inaktivierung der zugegebenen Nukleasen führt. Freigesetzte Erreger-DNA wurde mit Phenol/Chloroform extrahiert und mit 1 Isovolumen Isopropanol gefällt. Die gefällten Nukleinsäuren wurden mit 80% Ethanolol gewaschen und in 20 µl H₂O dest. aufgenommen.

### Beispiel 2

### Infektion mit Vacciniavirus und Serums

Rekombinantes Vacciniavirus mit dem Glykoprotein des Vesikulären Stomatitis Virus (VaccG) (Mackett et al.(1985), Science 227, 433-435) wurde auf BSC40 Zellen gezüchtet und die Viruskonzentration im Plaque-Assay bestimmt. C57BL/6 Mäuse (Institut für Labortierkunde, Universität Zürich) wurden i.v. mit 2 × 10⁶ pfu VaccG infiziert. Den Mäusen wurden 200-300 µl Blut an den Tagen 8, 16 und 30 nach Infektion entnommen und Serum wurde durch Zentrifugation gewonnen und bei -20°C gelagert.

Nach erfolgter Immunisierung der Mäuse wurde die erfolgreiche Induktion von anti-Vacciniaantikörpern im Neutralisationsassay gegen VSV getestet (Ludewig et al., 2000. Eur J Immunol, 30:185-196), um den besten Zeitpunkt der Serumabnahme für die geplanten Analysen zu ermitteln. Wie in Tabelle 1 dargestellt, hat ab Tag 16 der Anstieg sowohl des Gesamt-Immunglobulin, als auch der IgG-Klasse ihr Maximum erreicht, so dass dieses Serum der Abnahmetage 16 und 30 verwendet werden konnte.

**Tabelle 1: Titerverlauf von Antikörpern nach Immunisierung mit Vacciniavirus**

| **Tag post inoculationem** | **Gesamt-Immunglobulin (in 40xlog2)** | **IgG (in 40xlog2)** |
|---|---|---|
| 8 | 9 | 6 |
| 16 | 12 | 11 |
| 30 | 12 | 12 |

### Beispiel 3A Globale Amplifikation geringster Mengen an Erreger-Nukleinsäuren

Die globale Amplifikation von genomischen Nukleinsäuren des Erregers ist ein essentieller Schritt in dem erfindungsgemäßen Verfahren. Hierbei ist die Hauptherausforderung die sehr geringe Menge genonischer Keimnukleinsäuren, die (ohne Vorkultur) aus infizierten Geweben isoliert werden, umfassend (d.h. möglichts alle Abschnitte des Genoms miteinschliessend) zu amplifizieren. Zudem muss die amplifizierte DNA für das nachfolgende Screening exprimierbar und klonierbar sein. Während PCR-amplifizierte cDNA-Expressionsbibliotheken vielfach beschrieben, hergestellt und benutzt werden (z.B. Edwards et al., 1991) und z.T. als Kitlösung angeboten werden (z.B. SMART-cDNA library construction kit, Clontech), ist die Herstellung umfassender genomischer Bibliotheken ausgehend von geringen Mengen (<10 ng) von Erregernukleinsäuren bisher nicht beschrieben. Daher war es notwendig, für das erfindungsgemäße Verfahren ein DNA-Amplifikationsmodul zu entwickeln, welche die Herstellung von umfassenden genomischen Expressionsbanken aus Subnanogrammengen Material erlaubt. Die Methode wurde für das Vacciniavirusgenom etabliert und ist ohne Modifikationen für alle DNA-kodierten Erreger und mit geringen Modifikationen auf RNA kodierte Pathogene übertragbar. Die Amplifikation der Vacciniavirus-DNA wurde durch eine Klenow-Enzym Reaktion eingeleitet, für die Adaptor-Oligonukleotide mit degeneriertem 3'-Ende für ein sequenzunabhängiges Priming nach dem Random-Prinzip verwendet wurden. Hierauf folgen sequentiell zwei PCR-Amplifikationen mit Oligonukleotiden, die der Adaptorsequenz entsprechen. In einer Reihe von unabhängig durchgeführten Experimenten sind die Bedingungen für eine besonders effiziente Amplifikation erarbeitet worden. Nach Optimierung der Methode wurden zur Bestimmung der Sensitivität verschiedene Mengen Vaccinia-Virus DNA (u.a. 25 ng, 1 ng, 200 pg, 20 pg, 2 pg) mit 2 pMol Adaptor-N(6) (GATGTAATACGAA[P2][P3]TTGGACTCATATANNNNNN) gemischt, 5 min bei 95°C denaturiert und anschließend auf Eis abgekühlt. N steht hierbei für den degenerierten Primeranteil. Nach Vorbereitung eines Reaktionsansatzes mit Klenow-Enzym (2 U), DNA-Polymerase-1 Puffer (10 mM Tris-HCl pH 7,5, 5 mM MgCl₂, 7,5 mM Dithiothreitol, 1 nMol dNTPs) wurde eine Primerverlängerung bei 37°C für 2 h durchgeführt. Anschließend erfolgte die Reinigung der durch Klenow-Polymerase elongierten Fragmente von den freien Adaptoroligonukleotiden über Standardtechniken. Jeweils 1/25 (d.h. 1 ng, 40 pg, 8 pg, 0,8 pg, 0,08pg) der mit Klenow-Polymerase getaggten DNA wurden für einen ersten Amplifikationsschrittt eingesetzt. Die PCR-Amplifikation mit Adaptor-Oligonukleotiden wurde mit zwei unterschiedlichen Oligonukleotiden (EcoR1-Adaptoroligonukleotid GATGTAATACGAATTCGACTCATAT bzw. Mfe1-Adaptor-Oligonukleotid GATGTAATACAATTGGACTCATAT) (Anlagerung bei 60°C 1 min; Verlängerung bei 72°C. 2,5 min; Denaturierung 94°C 1min; 35 Zyklen) durchgeführt. Die einmalige Amplifikation der Nukleinsäuren bei Nukleinsäuren von weniger als 40 pg erwies sich als nicht ausreichend um einen optisch im Ethidiumbromid/Agarosegel detektierbaren Amplißkationsschmier zu erzeugen. Je 1 µl des Amplifikats wurden daher anschließend als Template in eine zweite Amplifikation unter identischen Bedingungen für 30-35 Zyklen überführt. Die amplifizierten Produkte wurden gelelektrophoretisch analysiert. Die beschriebenen PCR-Bedingungen führten zu einer Amplifikation mit Fragmenten unterschiedlicher Länge (150-2000 bp) in allen Versuchsanordnungen bis minimal 0,8 pg Template DNA (Abb. 2A). Dabei wurden bei niedrigeren Ausgangsmengen von DNA im Durchschnitt kürzere Fragmente amplifiziert. In verschiedenen Experimenten wurden die Bedingungen für die Re-PCR variiert. Hierbei erwies sich, dass eine Variation der Pufferbedingungen (z.B. Mg-Konzentration) und Variation der eingesetzten Enyzme z.B. Stoffel-Fragment der Taq-Polymerase unterschiedliche Amplifikationsmuster erzeugten (s. Abb. 6). Für die Reamplifikation werden lediglich 1/50 der initialen PCR verwendet. Somit kann die Re-Amplifikation unter 50 verschiedenen Bedingungen durchgeführt werden. Dass eine Variation der Amplifikationsbedingungen eine besonders gute umfassende globale Amplifikation erlauben, wurde in der Repräsentanz-Analyse im reversen Southern-Blot nachgewiesen (s. Abb. 6). Zur Überprüfung der Identität amplifizierter Fragmente wurden diese über Standardverfahren in den Bluescript Klonierungsvektor (Stratagene) ligiert und 20 Klone wurden sequenziert. 20 von 20 Sequenzen stimmten mit Vacciniavirus-Sequenzen überein, sodass eine Amplifikation von Artefakt-Sequenzen (z.B. polymerisierte Primersequenzen) ausgeschlossen werden konnte.

### Beispiel 3B: Herstellung einer genomischen Bibliothek

Die Herstellung einer Vaccinia-Bibliothek erfolgte durch Amplifikation von 20 pg einer mit Klenow-Polymerase getaggten Vaccinia DNA analog den im Beispiel 3A durchgeführten Bedingungen. Für die PCR-Amplifikation mit Adaptor-Oligonukleotiden wurden in getrennten Ansätzen mit zwei unterschiedlichen Oligonukleotiden (EcoR1-Adaptoroligonukleotid GATGTAATACGAATTCGACTCATAT bzw. Mfe1-Adaptor-Oligonukleotid GATGTAATACAATTGGACTCATAT) jeweils 1/50 der aufgereinigten Fragmente (Anlagerung bei 60°C 1 min, Verlängerung bei 72°C 2,5 min; Denaturierung 94°C 1min; 35-40 Zyklen) verwendet. Je 1 µl des Amplifikats wurden anschließend als Template in eine zweite Amplifikation unter identischen Bedingungen für 30 Zyklen überführt. Die amplifizierten Produkte wurden gelelektrophoretisch analysiert. Die beschriebenen PCR-Bedingungen führten zu einer Amplifikation mit Fragmenten unterschiedlicher Länge (200-2500bp) (Abb. 2B). Alle Kontrollreaktionen, in denen kein DNA-Template eingesetzt wurde, blieben negativ. Anschließend wurden die amplifizierten Produkte aufgereinigt, mit EcoR1- bzw. Mfe1-Restriktionsenzymen verdaut und in Lambda-ZAP-Express-Vektor (EcoR1-Fragment, Stratagene) ligiert. Die Kombination zweier unabhängiger Restriktionsenzyme erhöht die Diversifikation und die Wahrscheinlichkeit, dass immundominante Bereiche nicht durch interne Restriktionsenzymschnittstellen zerstört werden und damit dem Nachweis entgehen. Nach Ligation der Nukleinsäurefragmente in die Vektoren wurden diese nach Standardtechniken in Lambda-Phagen verpackt. Dies erfolgte mit kömmerziell erwerblichen Packaging Extrakten entsprechend den Herstellerangaben (z.B. Gigapack Gold III, Stratagene). Die so entstandenen Lambdaphagen-Bibliotheken (SE mit EcoRI-Adaptoren, SM für MfeI-Adaptoren) wurden ohne weitere Amplifikation im Immunoscreening analysiert.

### Beispiel 4

### Immunoscreening und Identifikation von Antigenen

Das Immunoscreening wurde wie bereits in Sahin et al. (1995), Proc Natl Acad Sci USA 92: 11810-3; und Türeci et al. (1997), Mol Med Today 3, 342-349 beschrieben durchgeführt.

Bakterien des *E.Coli* K12-abgeleiteten Stammes XL1 MRF wurden in der exponentiellen Wachstumsphase geerntet, auf eine OD₆₀₀=0,5 eingestellt und mit den Lambdaphagen der beschriebenen Expressionsbank infiziert. Die Anzahl der Plaque-bildenden Einheiten (plaqueforming-units, pfu) wurde derart eingestellt, dass eine Subkonfluenz der Plaques vorlag (z.B. ∼5000 pfu/ 145 mm Petrischale). Unter Zusatz von TOP-Agar und IPTG wurde der Infektionsansatz auf Agarplatten mit Tetrazyklin ausplattiert. Bei der Übernachtkultur bei 37°C bildeten sich auf dem Bakterienrasen Phagenplaques. Jeder einzelne Plaque repräsentiert einen Lambda-Phagen-Klon mit der in diesen Klon inserierten Nukleinsäure und enthält gleichzeitig das von der Nukleinsäuren kodierte rekombinant exprimierte Protein.

Nitrozellulosemembranen (Schleicher & Schüll) wurden aufgelegt, um Abklatschpräparate der rekombinanten Proteine herzustellen (Plaque Lift). Nach Waschschritten in TBS-Tween und Blocken unspezifischer Bindungsstellen in TBS + 10% Milchpulver erfolgte die Inkubation im Serum des infizierten Wirtes über Nacht. Es wurde zu diesem Zweck gepooltes Serum der Infektionstage 16 und 30 verwendet und 1:100 - 1: 1.000 verdünnt. Nach weiteren Waschschritten wurden die Nitrozellulose-Membranen mit einem gegen Maus-IgG gerichteten, sekundären AP-konjugierten Antikörper inkubiert. Bindungen von Serumantikörpern an in Phagenplaques rekombinant exprimierte Proteine konnten auf diese Weise durch eine Farbreaktion sichtbar gemacht werden. Klone, die als reaktiv mit Wirtsseren identifiziert wurden, konnten auf die Kulturplatte zurückverfolgt und von dort das entsprechende Phagenkonstrukt monoklonal isoliert werden. Nach erneuter Ausplattierung wurden solche positiven Klone bestätigt. Durch *in vivo* Exzision wurde der Lambdaphagenklon zum Phagemid rezirkularisiert (Sahin et al. (1995), Proc Natl Acad Sci USA 92: 11810-3).

In beiden Banken (SE und SM) wurden wie oben geschildert insgesamt 150.000 Klone gescreent. Zu diesem Zweck wurde das gepoolte Serum der infizierten Tiere vom Tag 16 und 30 nach Infektion in einer Verdünnung von 1:500 verwendet. Primär identifizierte Klone wurden zunächst oligoklonal unter Zunahme von benachbarten nicht-reaktiven Phagenplaques isoliert und nach Bestätigung monoklonalisiert (Abb. 3). Es konnten 26 (SE-Bank) bzw. 41 (SM-Bank) Klone isoliert werden, die mit dem Serum der immunisierten Tiere reaktiv waren.

Alle identifizierten Klone wurden zusätzlich mit Präimmunseren von Mäusen desselben Stammes getestet und waren nicht reaktiv.

**Tabelle 2: Anzahl reaktiver Klone nach Screening der SE- und SM-Bank**

| **Bank** | **gescreente Klone** | **reaktive Klone** |
|---|---|---|
| Bank SE | 150.000 | 26 |
| Bank SM | 150.000 | 41 |

Die Sequenzierung und der Datenbankvergleich deckte unter anderem die drei folgenden unterschiedlich immunogenen Vacciniavirusproteine unter den Klonen auf:

### 39 kDa Immunodominantes Antigen-Protein

Drei Klone kodieren für Bereiche aus dem 39-kDa Protein von Vacciniavirus (Abb. 4A). Die Klone stellen Fragmente dieses Proteins dar. Sie beginnen alle bei Nukleotid 288, reichen aber unterschiedlich weit an das 3'-Ende dieses Genproduktes, nämlich bis Nukleotid 688, 788 bzw. 938.

Das für das 39-kDa Protein kodierende Gen ist der ORF A4L im Western Reserve (WR)-Stamm (Maa und Esteban (1987), J. Virol. 61, 3910-3919). Das 281 Aminosäuren lange 39-kDa Protein ist in Menschen und Tieren stark immunogen (Demkovic et al. (1992), J. Virol. 66, 386-398).

Es wurde bereits beschrieben, dass eine Immunisierung mit dem 39-kDa Protein eine schützende Immunität in Mäusen induzieren kann (Demkovic et al. (1992), J. Virol. 66, 386-398).

Die stärkste antigene Domäne scheint innerhalb der letzten C-terminal gelegenen 103 Aminosäuren zu liegen (Demkovic et al: (1992), J.Virol. 66, 386-398). Die Lage der hier gefundenen Fragmente ist ebenfalls als Hinweis auf Sero-Epitope zu verstehen. Interessanterweise decken die beiden stark immunreaktiven Klone 2 und 3 den Bereich dieser als stark antigen beschriebenen 103 Aminosäuren ab.

An diesem Beispiel wird auch die Multidimensionalität der Aussagen des erfindungsgemäßen Verfahrens deutlich. Neben der Identifizierung des gleichzeitig auch Immunschutz gewährenden Antigens ist durch die Anzahl überlappender Klone ein Hinweis auf die Abundanz der Antikörper gegeben. Die Lage der Klone erlaubt eine Einengung der Sero-Epitope sowie die Stärke der Reaktivität einen Hinweis auf die Avidität der Antikörper. Dies gilt ebenso auch für die im folgenden beschriebenen Antigene.

### A-Type-Inclusion-Protein (ATI)

Einige der hier gefundenen Klone repräsentieren das A-Type-Inclusion-Protein (ATI) (Abb. 4B), ein ca. 160 kDa großes Protein bei verschiedenen Orthopoxviren (Patel et al. (1986), Virology 149, 174-189), das einen großen Anteil der Proteinmenge der charakteristischen Einschlusskörperchen ausmacht. Beim Vacciniavirus ist dieses Protein verkürzt und nur ca. 94 kDa groß (Amegadzie (1992), Virology 186, 777-782). ATI assoziiert spezifisch mit infektiösen intrazellulären reifen Vacciniapartikeln und ist nicht in behüllten extrazellulären Vacciniaviren zu finden (Uleato et al. (1996), J. Virol. 70, 3372-3377). ATI ist eines der immundominanten Antigene in Mäusen, wobei die immundominanten Domänen am Carboxyterminus des Moleküls liegen (Amegadzie et al. (1992), Virology 186, 777-782). Die drei hier gefundenen Klone, die identische Reaktionsstärken haben, decken den Bereich zwischen bp 308 und 1437 ab und sind entsprechend tatsächlich C-terminal bis zentral im kodierten Protein gelegen.

### Plaque size/host range (ps/hr)-Protein

Das 38 oder 45 kDa große Plaque size/host range-Protein (ps/hr) wird durch den ORF B5R kodiert (Takahashi-Nishimaki et al. (1991), Virology 181, 158-164). ps/hr ist ein Typ 1 Transmembranprotein, das in die Membran von extrazellulären Viruspartikeln inkorporiert wird oder von Zellen während der Infektion sezerniert werden kann. Antikörper gegen ps/hr neutralisieren die Infektiösität des Vacciniavirus (Galmiche et al. (1999), Virology 254, 71-80). Deletion von ps/hr führt zur Attenuierung des Virus *in vivo* (Stern et al. (1997), Virology 233, 118-129). Ausserdem schützt die Immunisierung mit B5R gegen eine Infektion mit ansonsten tödlichen Dosen des Virus (Galmiche et al. (1999), Virology 254, 71-80). Drei der hier identifizierten Klone stellen Fragmente dar, die wiederum denselben Bereich dieses Antigens abdecken und den C-Terminus einschließen (Abb. 4C). Dies bedeutet, dass das von diesen Klonen repräsentierte Sero-Epitop im extrazellulären Bereich dieses viralen Oberflächenmoleküls liegt und somit gut für Antikörper zugänglich ist.

### Beispiel 5

### Sequenzierung und bioinformatische Analyse der identifizierten Vacciniavirusantigene

Die Sequenzierung der identifizierten Klone erfolgte nach Standardtechniken mit Oligonukleotiden, die das Insert flankieren (BK-Reverse, BK-Universe) in Sanger'schen Kettenabbruchverfahren. Ermittelte Sequenzen wurden über BLAST-Analyse mit bekannten Sequenzen in der Genbank angeglichen. Die Lokalisation der Vacciniavirusantigene im Genom (Zugangsnummer M35027) und die Standardnomenklatur ist in Tabelle 3 angegeben. Diese Analyse zeigt, dass über das gesamte Vacciniavirusgenom verteilte Antigene mit dem erfindungsgemäßen Verfahren identifiziert wurden. Bei einer grossen Anzahl der identifizierten Gene ist bisher nicht bekannt gewesen, dass die Genprodukte eine Wirkung als Antigen besitzen. Mit Hilfe des erfindungsgemäßen Verfahrens werden daher nicht nur bekannte Antigene, sondern auch unbekannte Antigene identifiziert. Ein weiterer Vorteil des erfindungsgemäßen Verfahrens ist, dass Antigene identifiziert werden können, die auf beiden Strängen (kodierenden und komplementärer Strang) des Genoms zu finden sind.

**Tabelle 3: Identität, genomische Lokalisation, serologische Reaktivität und Anzahl der identifizierten Vacciniavirusantigene mit Hilfe des erfindungsgemäßen Verfahrens.**

| Vacciniavirus-Antigene | Lokalisation in VV-Genom | SEQ ID NO: | Signal | #Klon |
|---|---|---|---|---|
| 39 kDa immunodominantes Antigen (A4L) | ORF 151 (117270-116425) | 4 | +++ | 62 |
| 94 kDa A-Type-Inclusion-Protein (TA31L) | ORF 174 (138014-135837) | 5 | +++ | 30 |
| 33 kDa Plaque size/host range-Protein (B5R) | ORF 232 (167383-168336) | 6 | +++ | 7 |
| 116 kDa DNA-polymerase (E9L) | ORF 80 (59787-56767) | 7 | +++ | 4 |
| 65 kDa Hüle protein (F12L) | ORF 60 (43919-42012) | 8 | +++ | 2 |
| 62 kDa Prifampicin resistenzgen (D13L) | ORF 145 (113026-111371) | 9 | +++ | 1 |
| 32 kDa Carboanhydrase-ähnliches Protein (D8L) | ORF 137 (107120-106206) | 10 | +++ | 1 |
| 36 kDa late Protein (IIL) | ORF 87 (63935-62997) | 11 | +++ | 1 |
| 16 kDa Protein (TC14L) | ORF10 (10995-10567) | 12 | +++ | 1 |
| 38 kDa serinprotease-inhibitor 2 (B13R) | ORF 421 (172562-172912 | 13 | ++ | 4 |
| 18 kDa Protein (C7L) | ORF 24 (19157-18805) | 14 | ++ | 3 |
| 24.6 kDa Protein (B2R) | ORF 226 (163876-164535) | 15 | ++ | 2 |
| 36kDa Protein (A11R) | ORF 164 (124976-125932) | 16 | ++ | 1 |
| 15 kDa Membran-Phosphoprotein (A14L) | ORF 167 (126785-127128) | 17 | ++ | 1 |
| 147 kDa Protein (J6R) | ORF 117 (86510-90370) | 18 | ++ | 1 |
| 77kDa Protein(OIL)) | ORF 84 (62477-60477) | 19 | ++ | 1 |
| 59 kDa Protein (C2L) | ORF30 (24156-22618) | 20 | + | 4 |
| 90 kDa Protein (D5R) | ORF132 (101420-103777) | 21 | + | 1 |
| 23 kDa Protein (A17L) | ORF170 (129314-128703) | 22 | + | 1 |

In der Abbildung 5 ist eine grafische Repräsentation des Vacciniavirugenoms mit Darstellung der offenen Leseraster (ORF) zu finden, die zeigt, dass die durch das erfindungsgemäße Verfahren identifizierten Antigene über das gesamte Vacciniavirusgenom verteilt sind. Dies indiziert, dass das erfindungsgemäße Verfahren eine repräsentative Amplifikation von spezifischer Erregernukleinsäure aus geringsten Mengen von Ausgangsmaterial (1- 20 pg) erlaubt.

### Beispiel 6

### Reprätentstionsanalyse durch reversen Southern Blot

Die repräsentative Amplifikation aus geringsten Mengen von Erregernukleinsäuren durch das erfindungsgemäße Verfahren wurde ebenfalls im folgenden Experiment gezeigt.

Zehn Genabschnitte des Vacciniavirusgenoms wurden ausgewählt und durch PCR-Reaktionen amplifiziert. Nach gelelektrophoretischer Auftrennung wurden die DNA-Fragmente über alkalischen Transfer auf Nylonmembranen geblottet. Radioaktive Hybridisierung wurde mit 20 ng nach dem erfindungsgemäßen Verfahren hergestellter und mit ³²P markierter DNA durchgeführt. Abb. 6A zeigt, dass nur ein Teil der zehn zufällig ausgewählten Abschnitte des Genoms in der nach dem erfindungsgemäßen Verfahren hergestellten DNA in einer einzelnen Re-PCR DNA enthalten sind. Werden mehrere Re-PCR DNA, die in unterschiedlichen Ansätzen und unter variierten Bedingungen hergestellt wurden, kombiniert, ist die 100%ige Repräsentanz der zufällig ausgewählten Genabschnitte in der nach dem erfindungsgemäßen Verfahren hergestellten DNA offensichtlich (Abb. 6B). Die unterschiedliche Abundanz der Nukleinsäuren, z.B. des 39 kDa Antigens (Abb. 6B, Spur 10), kann zumindest teilweise erklären, dass bestimmte Genabschnitte häufiger in der nach dem erfindungsgemäßen Verfahren hergestellten DNA gefunden werden. Eine niedrige Abundanz der DNA im Amplifikat schliesst aber eine häufige Detektion im Screening nicht aus, wie das Beispiel der A-Type-Inclusion-Protein DNA (Abb. 6B, Spur 2) zeigt.

### Beispiel 7

### Differentielle Serologie

Lambdaphagen, deren rekombinante Inserts für Antigene kodierten, die durch Antikörper im Serum infizierter Mäuse erkannt werden, wurden auf Reaktivität mit Seren nicht infizierter Tiere (immunologisch naiv) und mit Seren von mit dem Lymphocytären Choriomeningitisvius infizierten Mäusen getestet. Diese Studien wurden ebenfalls als Plaque-Lift Assay durchgerührt. Der Antikörpertiter und die spezifische Reaktivität der Seren gegen die klonierten Antigene kann so einfach ermittelt werden. In Abb. 7A ist dargestellt, wie die Reaktivität eines am Tag 21 nach Infektion mit dem Vacciniavirus gewonnenen Serum gegen das 39 kDa Antigen ermittelt wurde. Zweifache Serumverdünnungen wurden mit durch Phagen in E. coli induziertem, rekombinantem 39 kDa Antigen inkubiert. Eine spezifische Reaktivität ist bei einer Serumverdünnung von 1:16000 noch erkennbar. Der zeitliche Verlauf der Antikörperreaktivität gegen das 39 kDa Antigen in mit Vacciniavirus infizierten, Lymphocytäres Choriomeningitisvirus-infizierten und in nicht infizierten Mäusen ist in Abb. 7B dargestellt. Der Verlauf der Antikörperantwort nach Vacciniavirusinfektion ist für diese Infektion typisch. Das Fehlen einer Reaktivität gegen das 39 kDa Antigen in naiven Mäusen und die nur sehr geringe Kreuzreaktivität nach Infektion mit dem Lymphocytären Choriomeningitisvirus zeigt die gute diagnostische Qualität, die mit durch das erfindungsgemäße Verfahren identifizierten Antigenen erreicht werden kann.

### Beispiel 8

### Identifikation von bakteriellen Antigenen mit Hilfe des erfindungsgemäßen Verfahrens

Zunächst wurden die Bedingungen erarbeitet, die eine Anreicherung von bakteriellen Erregern aus infizierten Proben erlauben. Hierzu wurde der Umstand genutzt, dass Bakterienwände widerstandsfähig gegenüber einer Lyse mit Solventien wie z.B. SDS sind. Durch eine SDS-Konzentrationsreihe wurde die Stabilität vom gramnegativen, grampositiven Bakterien und eukaryontischen Zellen bestimmt. Wie in Abbildung 8 dargestellt bleibt unter 1% SDS die korpuskuläre Struktur grampositiver Bakterien erhalten. Gramnegative Bakterien wie in diesem Beispiel E. coli weisen immerhin eine Widerstandsfähigkeit gegen Lyse bis 0,1% SDS auf. Dagegen werden alle Membranstrukturen (Zytoplasma, Kern) von eukaryontischen Zellen, wie in diesem Fall Fibroblasten, vollständig aufgelöst. Die hohe SDS-Sensitivität von eukaryontischen Zellen wurde in anderen Beispielen auch für Leukozyten, Milzzellen, und Lymphknotenbiopsien verifiziert. Nach Erarbeitung der Bedingungen wurde das erfindungsgemäße Verfahren für einen bisher nur unzureichend charakterisierten Erreger, Tropheryma whippelii, durchgeführt. Tropheryma whippelii ist ein gram-positives Bakterium und die Infektion mit diesem Erreger kann die Whipplesche Erkrankung auslösen. Die Whipplesche Erkrankung ist eine chronische Infektion verschiedener Organe mit der Hauptmanifestation im Darm, die unerkannt zum Tode führen kann. Dieser Erreger ist nur schwer in vitro anzüchtbar, so dass für molekulare Analysen nur geringste Mengen an spezifischer Nukleinsäure zur Verfügung stehen. Aufgrund dieser Probleme war die Analyse der antigenen Strukturen dieses Erregers bisher nicht möglich.

Durch das erfindungsgemäße Verfahren gelang es, potentielle Antigene dieses Erregers zu definieren. In der Abb. 9 sind die wesentlichen Schritte, die zur Charakterisierung von Tropheryma whippelii-spezifischen Antigenen geführt haben, dargestellt.

T. whippelii-haltiges, homogenisiertes Gehimmaterial eines an der Whippleschen Erkrankung verstorbenen Patienten wurde verwendet, um mit Interleukin-10 und Interleukin-4 deaktivierte Makrophagen zu inokulieren (Schoedon et al. (1997) J Infect Dis. 176:672-677). Am Tag 7 postokulationem wurden infizierte Makrophagen geerntet. und 25 µl des Makrophagen/Bakterien Gemischs wurden verarbeitet. Die differenzielle Zellyse erfolgte durch Inkubation der mit Bakterien infizierten Makrophagen in 1% SDS enhaltendem Proteinase K Puffer für 15 min mit 20 µg/ml Proteinase K bei 55°C. Durch diese Behandlung wurden die in der Mischung vorhandenen Makrophagen (eukaryontische Zellen) aufgelöst. Durch die Lyse der Makrophagen werden deren Nukleinsäuren (RNA, DNA) in die Lösung freigesetzt. Dagegen verbleiben die Nukleinsäuren der Bakterien durch Erhaltung der Integrität der grampositiven Hakterienwand in den Bakterienzellen. Nach der Inkubation mit SDS/Proteinase K wurden die Bakterien durch Zentrifugation der Suspension pelletiert. Dagegen ließen sich die Bakterien bei fehlendem Zusatz von Proteinase K aufgrund hoher Viskosität der Lösung schlechter pelletieren. Der Überstand mit den Nukleinsäuren der Makrophagen wurde verworfen und das kaum sichtbare Pellet mehrfach gewaschen. Nach den Waschschritten wurden pelletierte Bakterien in 100 µl Wasser resuspendiert und jeweis 10 µl der Suspension lichtmikroskopisch und nach Färbung mit dem DNA-Farbstoff DAPI in der Immunfluoreszens zur Bestimmung der Hakterienzahl verwendet. Die Anzahl der aus 25 µl der infizierten Makrophagen isolierten Bakterien betrug nach mikroskopischer Auszählung ca. 4000-6000 DNA-haltige Partikel. Die residualen 80 µl angereicherten Bakterien wurden zur Gewinnung von bakteriellen Nukleinsäuren nach Standardtechniken (Kochen, Denaturierung DNA-Isolation mit Phenol/Chloform) weiterverarbeitet. Die Menge der aus den Bakterien isolierten DNA konnte aufgrund der niedrigen Menge nicht experimentell quantifiziert werden (kein detektierbares Signal im EtBr-Gel). Aufgrund der licht- und immunfluoreszensmikroskopisch ermittelten Bakterienzahl (max. 6000) wurde bei einer angenommenen Bakteriengenomgrösse von 1-2 Mio Basen doppelsträngige DNA eine maximalen Ausbeute von 6-12 pg Erreger-DNA kalkuliert (Für die Kalkulation wurde das Durchschnittsgewicht eines Nukleotids von 660 eingesetzt). 50% der extrahierten DNA (d.h. max. 3-6 pg) wurde wie nach dem erfindungsgemäßen Verfahren beschrieben (Klenow, sequentielle PCR, Re-PCR) amplifiziert und aus den amplifizierten Fragmenten eine genomische Bibliothek in dem Lambda-ZAP-Express-Vektor (Stratagene) hergestellt. Das Immunoscreening wurde mit Seren von T. whippelii-infizierten Patienten durchgeführt. Positive Klone wurden sequenziert und bioinformatisch analysiert. Als Beispiel ist in Abb. 9 ein Klon aufgerührt der sowohl für ein bakterielles putatives Lipoprotein als auch für ein putatives Histidine Triad Protein kodiert.

Dieses Beispiel zeigt, dass das erfindungsgemäße Verfahren neben der Identifikation von viralen Antigenen auch geeignet ist, bakterielle Antigene zu identifizieren.

### Beispiel 9: Anreicherung von Whipple Bakterien aus einer infizierten Milzprobe eines Patienten mit systemischer Infektion.

Jeweils 20 µl einer kryopräservierten Milzprobe eines Patienten mit Morbus Whipple wurden unter fünf leicht modifizierten Bedingungen zur Anreicherung von Bakterien verwendet (insgesamt 100 µl). Hierzu wurden die Milzproben wie im Beispiel 8 beschrieben in 1,5 ml Proteinase K Puffer mit 20 mg/ml Proteinase K Zusatz 10-60 min bei 55°C inkubiert. Danach wurden die in den infizierten Milzprobe befindlichen Bakterien wie im obigen Beispiel beschrieben durch Zentrifugation angereichert und mikroskopisch wie vorher beschrieben dokumentiert. Abbildung 10 zeigt eine Aufnahme einer bakterienreichen Pelletfraktion. Anschliessend wurden die Bakterien durch Kochen in GITC-Puffer und Ultraschallbehandlung aufgeschlossen und die bakteriellen Nukleinsäuren über Standardverfahren isoliert. Wie erwartet, war die Menge der Nukleinsäuren, die aus den angereicherten Fraktionen isoliert wurden, unter der Nachweisgrenze von 1 ng. Zur Dokumentation der bakteriellen Anreicherung wurden jeweils 1/100 der isolierten Nukleinsäuren für eine PCR-Ampflifikation mit Whipple-Bakterien-bzw. human-DNA-spezifischen Oligonukleotiden eingesetzt und über 37 Zyklen amplifiziert. Abbildung 11A zeigt die Amplifikations-Resultate (A: PCR spezifisch für Whipple Bakterien, B: PCR spezifisch für humane DNA). Die Ergebnisse sind dargestellt für Amplifikationsbanden der Bakterien-angereicherten Fraktionen (Spur 1-3) bzw. der nicht-angereicherten Fraktionen (Spur 4-6). Während in den nicht-angereicherten Fraktionen wie erwartet die Amplifikationssignale für humane DNA deutlich stärker sind (Spur 4-6) zeigen besonders Fraktionen 1 und 2 fast ausschliesslich eine Amplfikation von Erregernukleinsäuren. Das Beispiel zeigt, dass die geringe Menge des benötigten Materials es ermöglicht, die Anreicherungsbedingungen leicht zu variieren und anschliessend mit de am stärksten angereicherten Erreger-Fraktion (in diesem Fall 1 und 2) das erfindungsgemäße Verfahren fortzusetzen.

### Beispiel 10: Globale Amplifikation von Erregernukleinsäuren aus direkt aus einer Milzprobe isolierten DNA

Die wie in Beispiel 9 isolierten Erregernukleinsäuren wurden wie in Beispiel 3 beschrieben amplifiziert und aus den amplifizierten Fragmenten eine genomische Bibliothek in Lambda-ZAP-Express-Vektor (Stratagene) hergestellt. Das Immunoscreening wurde mit Seren von T. whippelii-infizierten Patienten durchgeführt. Positive Klone wurden sequenziert und bioinformatisch analysiert.

## Patentansprüche

1. Verfahren zur Identifizierung von durch das Genom von mikrobiellen Erregern kodierten biologisch aktiven Strukturen ausgehend von genomischen Erregernukleinsäuren, umfassend die Schritte
(a) Gewinnung von genomischen Erregernukleinsäuren aus erregerhaltigen Proben,
(b) Sequenzunabhängige Amplifikation von genomischen Erregernukleinsäuren mittels Polymerase-Kettenreaktion oder reverser Transkription, oder beidem und unter Verwendung von Oligonukleotid-Primern mit Zufallssequenzen,
(c) Expression amplifizierter Erregernukleinsäuren, und
(d) Screening und Identifizierung der biologisch aktiven Struktur.

2. Verfahren gemäß Anspruch 1, **dadurch gekennzeichnet, dass** die durch das Genom von mikrobiellen Erregern kodierten biologisch aktiven Strukturen durch das Genom eines bakteriellen Erregen kodiert werden.

3. Verfahren gemäß Anspruch 1, **dadurch gekennzeichnet, dass** die durch das Genom von mikrobieller Erregern kodierten biologisch aktiven Strukturen durch das Genom eines DNA-haltigen Virus kodiert werden.

4. Verfahren gemäss den Ansprüchen 1-3, wobei der mikrobielle Erreger ein intrazellulärer viraler oder bakterieller Erreger ist.

5. Verfahren gemäss den Ansprüchen 1-3, wobei der mikrobielle Erreger ein extrazellulärer viraler oder bakterieller Erreger ist.

6. Verfahren gemäss den Ansprüchen 1-3, wobei der mikrobielle Erreger nicht-vital und/oder nicht-infektiös ist.

7. Verfahren gemäß Anspruch 1, wobei die biologisch aktive Struktur ein Erregerantigen ist.

8. Verfahrengemäß Anspruch 1, wobei die biologisch aktive Struktur ein Pathogenitätsfaktor des mikrobiellen Erregers ist.

9. Verfahren gemäß Anspruch 1, wobei die biologisch aktive Struktur ein enzymatisch aktives Protein ist.

10. Verfahren gemäss Anspruch 1, wobei zur Identifikation 10-20 pg Erregernukleinsäure verwendet werden.

11. Verfahren gemäss Anspruch 1, wobei zur Identifikation 1-10 pg Erregernukleinsäure verwendet werden.

12. Verfahren gemäss Anspruch 1, wobei die Proben Blut, Gewebe, kultivierte Zellen, Serum, Sekret aus Läsionen und andere Körperflüssigkeiten umfassen.

13. Verfahren gemäss Anspruch 1, wobei die Gewinnung von genomischen Erregernukleinsäuren aus erregerhaltigen Proben in Schritt (a) die folgenden Schritte umfasst:
(a₁) Freisetzung von Erregerpartikeln aus erregerhaltigen Proben
*optional* (a₂) Elimination und/oder Reduktion kontaminierender Wirtsnukleinsäuren
und (a₃) Extraktion der genomischen Erregernukleinsäure aus freigesetzten Erregerpartikeln.

14. Verfahren gemäss Anspruch 13, wobei die Freisetzung von Erregerpartikeln in Schritt (a₁) durch Zellyse, Sedimentation, Zentrifugation und/oder Filtration erfolgt.

15. Verfahren gemäss Anspruch 13, wobei die Elimination und/oder Reduktion kontaminierender Wirtsnukleinsäuren in Schritt (a₂) durch RNase- und/oder DNase-Verdau erfolgt.

16. Verfahren gemäss Anspruch 13, wobei die Extraktion der genomischen Erregernukleinsäure in Schritt (a₃) durch Abtrennung des genetischen Materials des Erregers von korpuskulären Bestandteilen des Erregers durch Proteinase K-Verdau, Denaturierung, Lysozym-Behandlung oder organische Extraktion erfolgt.

17. Verfahren gemäss Anspruch 1, wobei die Amplifikation der Erregernukleinsäure in Schritt (b) durch reverse Transkription mit degenerierten Oligonukleotiden und nachfolgende Amplifikation durch PCR erfolgt.

18. Verfahren gemäss Anspruch 1, wobei die Amplifikation der Erregernukleinsäure in Schritt (b) durch reverse Transkription mit degenerierten Oligonukleotiden und nachfolgende Amplifikation mit T7 RNA Polymerase erfolgt.

19. Verfahren gemäss Anspruch 1, wobei zur Expression von amplifizierten Erregernukleinsäuren in Schritt (c) ein Einbringen der Erregernukleinsäuren in Vektoren und Verpackung der Vektoren in Lambda-Phagen erfolgt.

20. Verfahren gemäss Anspruch 1, wobei zur Expression von amplifizierten Erregernukleinsäuren in Schritt (c) ein Einbringen der Erregernukleinsäuren in filamentöse Phagen-Vektoren erfolgt.

21. Verfahren gemäss den Ansprüchen 19 und 20, wobei die Vektoren ausgewählt sind aus der Gruppe der viralen, eukaryotischen oder prokaryotischen Vektoren.

22. Verfahren gemäss Anspruch 1, wobei das Screening ein Immunoscreening auf Erregerantigene darstellt und die Identifizierung von Erregerantigenen in Schritt (d) die folgenden Schritte umfasst:
(d₁) Infektion von Bakterien mit Lambda-Phagen,
(d₂) Kultivierung der infizierten Bakterien unter Bildung von Phagenplaques,
(d₃) Transfer der Phagenplaques auf eine Nitrozellulosemembran oder eine andere Festphase, die zur Immobilisierung von rekombinanten, aus den Erregern abgeleiteten Proteinen geeignet ist,
(d₄) Inkubation der Membran mit Serum oder antikörperhaltigen Körperflüssigkeiten des infizierten Wirtes,
(d₅) Waschen der Membran,
(d₆) Inkubation der Membran mit sekundärem AP-gekoppeltem anti-IgG-Antikörper, der spezifisch für Immunglobuline des infizierten Wirtes ist.
(d₇) Nachweis der mit Wirtsserum reaktiven Klone durch Farbreaktion, und
(d₈) Isolierung und Sequenzierung der reaktiven Klone.

23. Verfahren gemäss Anspruch 1, wobei das Screening ein Immunoscreening auf Erregerantigene darstellt und die Identifizierung von Erregerantigenen in Schritt (d) die folgenden Schritte umfasst:
(d₁) Generierung von rekombinanten filamentösen Phagen durch Einbringen der filamentösen Phagenvektoren in Bakterien,
(d₂) Inkubation generierter rekombinanter filamentöser Phagen mit Serum eines infizierten Wirtes,
(d₃) Selektion der filamentösen Phagen, an die Immunglobuline des Wirts gebunden haben, über immobilisierte Reagentien die spezifisch für die Immunglobuline des infizierten Wirts sind, und
(d₄) Isolierung und Sequenzierung der selektierten Klone.

24. Verfahren gemäss Anspruch 1, wobei der mikrobielle Erreger vor Gewinnung der Nukleinsäuren durch Fällung mit Polyethylenglykol, Ultrazentrifugation, Gradientenzentrifugation oder durch Affinitätschromatographie angereichert wird.

## Claims

1. A method for identifying biologically active structures encoded by the genome of microbial pathogens, using genomic pathogen nucleic acids, comprising the steps of:
(a) preparing genomic pathogen nucleic acids from pathogen-containing samples,
(b) amplifying in a sequence-independent manner genomic pathogen nucleic acids by polymerase chain reaction or reverse transcription or both using oligonucleotide primers having random sequences;
(c) expressing amplified pathogen nucleic acids and
(d) screening and identifying the biologically active structure.

2. The method according to Claim 1, **characterized in that** the biologically active structures encoded by the genome of microbial pathogens are encoded by the genome of a bacterial pathogen.

3. The method according to Claim 1, **characterized in that** the biologically active structures encoded by the genome of microbial pathogens are encoded by the genome of a DNA-containing virus.

4. The method according to Claims 1-3, wherein the microbial pathogen is an intracellular viral or bacterial pathogen.

5. The method according to Claims 1-3, wherein the microbial pathogen is an extracellular viral or bacterial pathogen.

6. The method according to Claims 1-3, wherein the microbial pathogen is non-vital and/or non-infectious.

7. The method according to Claim 1, wherein the biologically active structure is a pathogen antigen.

8. The method according to Claim 1, wherein the biologically active structure is a pathogenicity factor of the microbial pathogen.

9. The method according to Claim 1, wherein the biologically active structure is an enzymatically active protein.

10. The method according to Claim 1, wherein 10-20 pg of pathogen nucleic acid are used for identification.

11. The method according to Claim 1, wherein 1-10 pg of pathogen nucleic acid are used for identification.

12. The method according to Claim 1, wherein the samples comprise blood, tissue, cultured cells, serum, secretions from lesions, and other body fluids.

13. The method according to Claim 1, wherein preparing genomic pathogen nucleic acids from pathogen-containing samples in step (a) comprises the following steps:
(a₁) releasing pathogen particles from pathogen-containing samples,
optionally (a₂) eliminating and/or reducing contaminating host nucleic acids,
and (a₃) extracting the genomic pathogen nucleic acid from released pathogen particles.

14. The method according to Claim 13, wherein releasing pathogen particles in step (a₁) is carried out by cell lysis, sedimentation, centrifugation and/or filtration.

15. The method according to Claim 13, wherein eliminating and/or reducing contaminating host nucleic acids in step (a₂) is carried out by digestion using RNase and/or DNase.

16. The method according to Claim 13, wherein extracting the genomic pathogen nucleic acid in step (a₃) is carried out by separating the genetic material of the pathogen from corpuscular components of the pathogen by digestion using proteinase K, denaturation, lysozyme treatment or organic extraction.

17. The method according to Claim 1, wherein amplifying the pathogen nucleic acid in step (b) is carried out by reverse transcription using degenerated oligonucleotides and subsequent PCR amplification.

18. The method according to Claim 1, wherein amplifying the pathogen nucleic acid in step (b) is carried out by reverse transcription using degenerated oligonucleotides and subsequent amplification with T7 RNA polymerase.

19. The method according to Claim 1, wherein for expressing amplified pathogen nucleic acids in step (c) the pathogen nucleic acids are introduced into vectors and the vectors are packaged into lambda phages.

20. The method according to Claim 1, wherein for expressing amplified pathogen nucleic acids in step (c) the pathogen nucleic acids are introduced into filamentous phage vectors.

21. The method according to Claims 19 and 20, wherein the vectors are selected from the group consisting of viral, eukaryotic or prokaryotic vectors.

22. The method according to Claim 1, wherein the screening is an immunoscreening for pathogen antigens, and identifying pathogen antigens in step (d) comprises the following steps:
(d₁) infecting bacteria with lambda phages,
(d₂) culturing the infected bacteria to form phage plaques,
(d₃) transferring the phage plaques onto a nitrocellulose membrane or another solid phase suitable for immobilizing recombinant proteins derived from the pathogens,
(d₄) incubating the membrane with serum or antibody-containing body fluids of the infected host,
(d₅) washing the membrane,
(d₆) incubating the membrane with a secondary AP-coupled anti-IgG-antibody which is specific for immunoglobulins of the infected host, (d₇) detecting the clones reacting with host serum by colour reaction, and
(d₈) isolating and sequencing the reactive clones.

23. The method according to Claim 1, wherein the screening is an immunoscreening for pathogen antigens, and identifying pathogen antigens in step (d) comprises the following steps:
(d₁) generating recombinant filamentous phages by introducing the filamentous phage vectors into bacteria,
(d₂) incubating generated recombinant filamentous phages with serum from an infected host,
(d₃) selecting the filamentous phages to which host immunoglobulins have bound using immobilized reagents which are specific for the immunoglobulins of the infected host, and
(d₄) isolating and sequencing the selected clones.

24. The method according to Claim 1, wherein the microbial pathogen prior to preparing the nucleic acids is enriched by precipitation with polyethylene glycol, ultracentrifugation, gradient centrifugation or affinity chromatography.

## Revendications

1. Procédé pour l'identification de structures biologiquement actives codées par le génome d'agents pathogènes microbiens à partir d'acides nucléiques d'agents pathogènes génomiques, comprenant les étapes de:
(a) obtention d'acides nucléiques d'agents pathogènes génomiques à partir d'échantillons contenant des agents pathogènes,
(b) amplification indépendante des séquences d'acides nucléiques d'agents pathogènes génomiques au moyen d'une réaction d'amplification en chaîne par polymérase ou d'une réverse transcription, ou des deux, et avec utilisation d'amorces oligonucléotidiques ayant des séquences aléatoires,
(c) expression des acides nucléiques d'agents pathogènes amplifiés, et
(d) criblage et identification de la structure biologiquement active.

2. Procédé selon la revendication 1, **caractérisé en ce que** les structures biologiquement actives codées par le génome d'agents pathogènes microbiens sont codées par l'intermédiaire du génome d'un agent pathogène bactérien.

3. Procédé selon la revendication 1, **caractérisé en ce que** les structures biologiquement actives codées par le génome d'agents pathogènes microbiens sont codées par l'intermédiaire du génome d'un virus à ADN.

4. Procédé selon les revendications 1-3, dans lequel l'agent pathogène microbien est un agent pathogène viral ou bactérien intracellulaire.

5. Procédé selon les revendications 1-3, dans lequel l'agent pathogène microbien est un agent pathogène viral ou bactérien extracellulaire.

6. Procédé selon les revendications 1-3, dans lequel l'agent pathogène microbien est non-vital et/ou non-infectieux.

7. Procédé selon la revendication 1, dans lequel la structure biologiquement active est un antigène d'agent pathogène.

8. Procédé selon la revendication 1, dans lequel la structure biologiquement active est un facteur de pathogénicité de l'agent pathogène microbien.

9. Procédé selon la revendication 1, dans lequel la structure biologiquement active est une protéine à activité enzymatique.

10. Procédé selon la revendication 1, dans lequel on utilise, pour l'identification, 10-20 pg d'acide nucléique d'agent pathogène.

11. Procédé selon la revendication 1, dans lequel on utilise, pour l'identification, 1-10 pg d'acide nucléique d'agent pathogène.

12. Procédé selon la revendication 1, dans lequel les échantillons comprennent du sang, des tissus, des cellules cultivées, du sérum, des sécrétions provenant de lésions et d'autres fluides corporels.

13. Procédé selon la revendication 1, dans lequel l'obtention d'acides nucléiques d'agents pathogènes génomiques à partir d'échantillons contenant des agents pathogènes dans l'étape (a), comprend les étapes suivantes:
(a₁) libération de particules d'agent pathogène à partir d'échantillons contenant des agents pathogènes en option, (a₂) élimination et/ou réduction des acides nucléiques hôtes contaminants,
et (a₃) extraction de l'acide nucléique de l'agent pathogène génomique à partir des particules d'agent pathogène libérées.

14. Procédé selon la revendication 13, dans lequel la libération de particules d'agent pathogène dans l'étape (a₁) s'effectue par lyse cellulaire, sédimentation, centrifugation et/ou filtration.

15. Procédé selon la revendication 13, dans lequel l'élimination et/ou la réduction d'acides nucléiques hôtes contaminants dans l'étape (a₂) s'effectue par digestion de RNase et/ou de DNase.

16. Procédé selon la revendication 13, dans lequel l'extraction de l'acide nucléique génomique dans l'étape (a₃) s'effectue par séparation du matériel génétique de l'agent pathogène des constituants corpusculaires de l'agent pathogène par digestion par protéinase K, dénaturation, traitement par lysozyme ou extraction organique.

17. Procédé selon la revendication 1, dans lequel l'amplification de l'acide nucléique d'agent pathogène dans l'étape (b) s'effectue par réverse transcription avec des oligonucléotides dégénérés et amplification subséquente par ACP.

18. Procédé selon la revendication 1, dans lequel l'amplification de l'acide nucléique d'agent pathogène dans l'étape (b) s'effectue par réverse transcription avec des oligonucléotides dégénérés et amplification subséquente avec une ARN polymérase de T7.

19. Procédé selon la revendication 1, dans lequel pour l'expression des acides nucléiques d'agents pathogènes amplifiés dans l'étape (c) on met en oeuvre une introduction des acides nucléiques d'agents pathogènes dans des vecteurs et une encapsulation des vecteurs dans des phages lambda.

20. Procédé selon la revendication 1, dans lequel pour l'expression des acides nucléiques d'agents pathogènes amplifiés dans l'étape (c) on met en oeuvre une introduction des acides nucléiques d'agents pathogènes dans des vecteurs de phages filamenteux.

21. Procédé selon les revendications 19 et 20, dans lequel les vecteurs sont choisis dans le groupe des vecteurs viraux, eucaryotes ou procaryotes.

22. Procédé selon la revendication 1, dans lequel le criblage constitue un criblage immunologique sur des antigènes d'agents pathogènes et l'identification d'antigènes d'agents pathogènes dans l'étape (d) comprend les étapes suivantes:
(d₁) infection de bactéries avec des phages lambda,
(d₂) culture des bactéries infectées avec formation de plaques de phages,
(d₃) transfert des plaques de phages sur une membrane de nitrocellulose ou une autre phase solide, convenant pour l'immobilisation de protéines recombinantes extraites des agents pathogènes,
(d₄) incubation de la membrane avec du sérum ou des fluides corporels de l'hôte infecté contenant des anticorps,
(d₅) lavage de la membrane,
(d₆) incubation de la membrane avec un anticorps anti-IgG secondaire couplé à de l'AP, qui est spécifique pour les immunoglobulines de l'hôte infecté,
(d₇) détermination des clones réactifs avec le sérum de l'hôte par réaction colorée, et
(d₈) isolement et séquençage des clones réactifs.

23. Procédé selon la revendication 1, dans lequel le criblage constitue un criblage immunologique sur des antigènes d'agents pathogènes et l'identification d'antigènes d'agents pathogènes dans l'étape (d) comprend les étapes suivantes:
(d₁) génération de phages filamenteux recombinants par introduction des vecteurs de phages filamenteux dans des bactéries,
(d₂) incubation des phages filamenteux recombinants générés avec du sérum d'un hôte infecté,
(d₃) sélection des phages filamenteux auxquels se sont liées les immunoglobulines de l'hôte, par l'intermédiaire des réactifs immobilisés qui sont spécifiques pour les immunoglobulines de l'hôte infecté,
(d₄) isolement et séquençage des clones sélectionnés.

24. Procédé selon la revendication 1, dans lequel l'agent pathogène microbien est enrichi, avant l'obtention des acides nucléiques, par précipitation avec du polyéthylèneglycol, ultracentrifugation, centrifugation avec gradients ou par chromatographie d'affinité.


### SEQUENZ PROTOKOLL

<110> Ugur Sahin, özlem Türeci, Burkhard Ludewig
<120> Verfahren zur Identifizierung biologisch aktiver Strukturen mikrobieller Erreger
<130> 268-3 PCT
<150> DE 10108626.1
   <151> 2001-02-22
<160> 22
<170> PatentIn Ver. 2.1
<210> 1
   <211> 32
   <212> DNA
   <213> Künstliche Sequenz
<220>
   <223> Beschreibung der künstlichen Sequenz: Adaptor-N(6)-Oligonukleotide
<400> 1
   gatgtaatac gaattggact catatannnn nn 32
<210> 2
   <211> 25
   <212> DNA
   <213> Künstliche Sequenz
<220>
   <223> Beschreibung der künstlichen Sequenz: EcoRI-Adaptor-Oligonukleotide
<400> 2
   gatgtaatac gaattcgact catat 25
<210> 3
   <211> 24
   <212> DNA
   <213> Künstliche Sequenz
<220>
   <223> Beschreibung der künstlichen Sequenz: Mfel-Adaptor-Oligonukleotide <400> 3

   gatgtaatac aattggactc atat 24
<210> 4
   <211> 846
   <212> DNA
   <213> Vaccinia virus
<400> 4
   ttacttttgg aatcgttcaa aacctttgac tagttgtaga atttgatcta ttgccctacg 60
   cgtatactcc cttgcatcat atacgttcgt caccagatcg tttgtttcgg cctgaagttg 120
   gtgcatatct ttttcaacac tcgacatgag atccttaagg gccatatcgt ctagattttg 180
   ttgagatgct gctcctggat ttggattttg ttgtgctgtt gtacatactg taccaccagt 240
   aggtgtagga gtacatacag tggccacaat aggaggttga ggaggtgtaa ccgttggagt 300
   agtacaagaa atatttccat ccgattgttg tgtacatgta gttgttggta acgtctgaga 360
   aggttgggta gatggcggcg tcgtcgtttt ttgatcttta ttaaatttag agataatatc 420
   ctgaacagca ttgctcggcg tcaacgctgg aaggagtgaa ctcgccggcg catcagtatc 480
   ttcagacagc caatcaaaaa gattagacat atcagatgat gtattagttt gttgtcgtgg 540
   ttttggtgta ggagcagtac tactaggtag aagaatagga gccggtgtag ctgttggaac 600
   cggctgtgga gttatatgaa tagttggttg tagcggttgg ataggctgtc tgctggcggc 660
   catcatatta tctctagcta gttgttctcg caactgtctt tgataatacg actcttgaga 720
   ctttagtcct atttcaatcg cttcatcctt tttcgtatcc ggatcctttt cttcagaata 780
   atagattgac gactttggtg tagaggattc tgccagcccc tgtgagaact tgttaaagaa 840
   gtccat                                                            846
<210> 5
   <211> 2178
   <212> DNA
   <213> Vaccinia virus
<400> 5
   ctaagacgtc gcatctctct ctgtttcggc attggtttca ttattacgtc tacagtcgtt 60
   caactgtctt tcaagatctg atattctaga ttggagtctg ctaatctctg tagcattttc 120
   acggcattca ctcagttgtc tttcaagatc tgagatttta gattggagtc tgctaatctc 180
   tgtaagattt cctcctccgc tctcgatgca gtcggtcaac ttattctcta gttctctaat 240
   acgcgaacgc agtgcatcaa cttcttgcgt gtcttcctgg ttgcgtgtac attcatcgag 300
   tctagattcg agatctctaa cgcgtcgtcg ttcttcctca agttctctgc gtactacaga 360
   aagcgtgtcc ctatcttgtt gatatttagc aatttctgat tctagagtac tgattttgct 420
   tacgtagtta ctaatatttg tcttggcctt atcaagatcc tccttgtatt tgtcgcattc 480
   cttgatatcc ctacgaagtc tggacagttc ccattcgaca ttacgacgtt tatcgatttc 540
   agctcggaga tcgtcatcgc gttgttttag ccacatacga ctgagttcaa gttctcgttg 600
   acaagatcca tctacttttc cattcctaat agtatccagt tccttttcta gttctgaacg 660
   catttctcgt tccctatcaa gcgattctct caattctcgg atagtcttct tatcaatttc 720
   taataaatct gaaccatcat ctgtcccatt ttgaatatcc ctgtgttctt tgatctcttt 780
   tgtaagtcgg tcgattcttt cggttttata aacagaatcc ctttccaaag tcctaatctt 840
   actgagttta tcactaagtt ctgcattcaa ttcggtgagt tttctcttgg cttcttccaa 900
   ctctgtttta aactctccac tatttccgca ttcttcctcg catttatcta accattcaat 960
   tagtttatta ataactagtt ggtaatcagc gattcctata gccgttcttg taattgtggg 1020
   aacataatta ggatcttcta atggattgta tggcttgata gcatcatctt tatcattatt 1080
   agggggatgg acaaccttaa ttggttggtc ctcatctcct ccagtagcgt gtggttcttc 1140
   aataccagtg ttagtaatag gcttaggcaa atgcttgtcg tacgcgggca cttcctcatc 1200
   catcaagtat ttataatcgg gttctacttc agaatattct tttctaagag acgcgacttc 1260
   gggagttagt agaagaactc tgtttctgta tctatcaacg ctggaatcaa tactcaagtt 1320
   aaggatagcg aatacctcat cgtcatcatc cgtatcttct gaaacaccat catatgacat 1380
   ttcatgaagt ctaacgtatt gataaataga atcagattta gtattaaaca gatccttaac 1440
   ctttttagta aacgcatatg tatattttag atctccagat ttcataatat gatcacatgc 1500
   cttaaatgtc agtgcttcca tgatataatc tggaacacta atgggtgatg aaaaagatac 1560
   cggaccatat gctacgttga taaataactc tgaaccacta agtagataat gattaatgtt 1620
   aaggaagagg aaatattcag tatataggta tgtcttggcg tcatatcttg tactaaacac 1680
   gctaaacagt ttgttaatgt gatcaatttc caatagatta attagagcag caggaatacc 1740
   aacaaacata ttaccacatc cgtattttct atgaatatca catatcatgt taaaaaatct 1800
   tgatagaaga gcgaatatct cgtctgactt aatgagtcgt agttcagcag caacataagt 1860
   cataactgta aatagaacat actttcctgt agtattgatt ctagactccg catcaacacc 1920
   attattaaaa atagttttat atacatcttt aatctgctct ccgttaatcg tcgaacgttc 1980
   tagtatacgg aaacactttg atttcttatc tgtagttaat gacttagtga tatcacgaag 2040
   aatattacga attacatttc ttgtttttct tgagagacat gattcagaac tcaactcatc 2100
   gttccatagt ttttctacct cagtggcgaa atctttggag tgcttggtac atttttcaat 2160
   aaggttcgtg acctccat 2178
<210> 6
   <211> 954
   <212> DNA <213> Vaccinia virus
<400> 6
   atgaaaacga tttccgttgt tacgttgtta tgcgtactac ctgctgttgt ttattcaaca 60
   tgtactgtac ccactatgaa taacgctaaa ttaacgtcta ccgaaacatc gtttaataat 120
   aaccagaaag ttacgtttac atgtgatcag ggatatcatt cttcggatcc aaatgctgtc 180
   tgcgaaacag ataaatggaa atacgaaaat ccatgcaaaa aaatgtgcac agtttctgat 240
   tacatctctg aactatataa taaaccgcta tacgaagtga attccaccat gacactaagt 300
   tgcaacggcg aaacaaaata ttttcgttgc gaagaaaaaa atggaaatac ttcttggaat 360
   gatactgtta cgtgtcctaa tgcggaatgt caacctcttc aattagaaca cggatcgtgt 420
   caaccagtta aagaaaaata ctcatttggg gaatatatga ctatcaactg tgatgttgga 480
   tatgaggtta ttggtgcttc gtacataagt tgtacagcta attcttggaa tgttattcca 540
   tcatgtcaac aaaaatgtga tataccgtct ctatctaatg gattaatttc cggatctaca 600
   ttttctatcg gtggcgttat acatcttagt tgtaaaagtg gttttatact aacgggatct 660
   ccatcatcca catgtatcga cggtaaatgg aatcccgtac tcccaatatg tgtacgaact 720
   aacgaagaat ttgatccagt ggatgatggt cccgacgatg agacagattt gagcaaactc 780
   tcgaaagacg ttgtacaata tgaacaagaa atagaatcgt tagaagcaac ttatcatata 840
   atcatagtgg cgttaacaat tatgggcgtc atatttttaa tctccgttat agtattagtt 900
   tgttcctgtg acaaaaataa tgaccaatat aagttccata aattgctacc gtaa       954
<210> 7
   <211> 3021
   <212> DNA
   <213> Vaccinia virus
<400> 7
   ttatgcttcg taaaatgtag gttttgaacc aaacattctt tcaaagaatg agatgcataa 60
   aactttatta tccaatagat tgactatttc ggacgtcaat cgtttaaagt aaacttcgta 120
   aaatattctt tgatcactgc cgagtttaaa acttctatcg ataattgtct catatgtttt 180
   aatatttaca agttttttgg tccatggtac attagccgga caaatatatg caaaataata 240
   tcgttctcca agttctatag tttctggatt atttttatta tattcagtaa ccaaatacat 300
   attagggtta tctgcggatt tataatttga gtgatgcatt cgactcaaca taaataattc 360
   tagaggagac gatctactat caaattcgga tcgtaaatct gtttctaaag aacggagaat 420
   atctatacat acctgattag aattcatccg tccttcagac aacatctcag acagtctggt 480
   cttgtatgtc ttaatcatat tcttatgaaa cttggaaaca tctcttctag tttcactagt 540
   acctttatta attctctcag gtacagattt tgaattcgac gatgctgagt atttcatcgt 600
   tgtatatttc ttcttcgatt gcataatcag attcttatat accgcctcaa actctatttt 660
   aaaattatta aacaatactc tattattaat cagtcgttct aactctttcg ctatttctat 720
   agacttatcg acatcttgac tgtctatctc tgtaaacacg gagtcggtat ctccatacac 780
   gctacgaaaa cgaaatctgt aatctatagg caacgatgtt ttcacaatcg gattaatatc 840
   tctatcgtcc atataaaatg gattacttaa tggattggca aaccgtaaca taccgttaga 900
   taactctgct ccatttagta ccgattctag atacaagatc attctacgtc ctatggatgt 960
   gcaactctta gccgaagcgt atgagtatag agcactattt ctaaatccca tcagaccata 1020
   tactgagttg gctactatct tgtacgtata ttgcatggaa tcatagatgg ccttttcagt 1080
   tgaactggta gcctgtttta acatcttttt atatctggct ctctctgcca aaaatgttct 1140
   taatagtcta ggaatggttc cttctatcga tctatcgaaa attgctattt cagagatgag 1200
   gttcggtagt ctaggttcac aatgaaccgt aatatatcta ggaggtggat atttctgaag 1260
   caatagctga ttatttattt cttcttccaa tctattggta ctaacaacga caccgactaa 1320
   tgtttccgga gatagatttc caaagataca cacattagga tacagactgt tataatcaaa 1380
   gattaataca ttattactaa acattttttg ttttggagca aataccttac cgccttcata 1440
   aggaaacttt tgttttgttt ctgatctaac taagatagtt ttagtttcca acaatagctt 1500
   taacagtgga cccttgatga ctgtactcgc tctatattcg aataccatgg attgaggaag 1560
   cacatatgtt gacgcacccg cgtctgtttt tgtttctact ccataatact cccacaaata 1620
   ctgacacaaa caagcatcat gaatacagta tctagccata tctaaagcta tgtttagatt 1680
   ataatcctta tacatctgag ctaaatcaac gtcatccttt ccgaaagata atttatatgt 1740
   atcattaggt aaagtaggac ataatagtac gactttaaat ccatttcccc aaatatcttt 1800
   acgaattact ttacatataa tatcctcatc aacagtcaca taattacctg tggttaaaac 1860
   ctttgcaaat gcagcggctt tgcctttcgc gtctgtagta tcgtcaccga taaacgtcat 1920
   ttctctaact cctctattta atactttacc catgcaactg aacgcgttct tggatataga 1980
   atccaatttg tacgaatcca atttttcaga tttttgaatg aatgaatata gatcgaaaaa 2040
   tatagttcca ttattgttat taacgtgaaa cgtagtattg gccatgccgc ctactccctt 2100
   atgactagac tgatttctct cataaataca gagatgtaca gcttcctttt tgtccggaga 2160
   tctaaagata atcttctctc ctgttaataa ctctagacga ttagtaatat atctcagatc 2220
   aaagttatgt ccgttaaagg taacgacgta gtcgaacgtt agttccaaca attgtttagc 2280
   tattcgtaac aaaactattt cagaacatag aactagttct cgttcgtaat ccatttccat 2340
   tagtgactgt atcctcaaac atcctctatc gacggcttct tgtatttcct gttccgttaa 2400
   catctcttca ttaatgagcg taaacaataa tcgtttacca cttaaatcga tataacagta 2460
   acttgtatgc gagattgggt taataaatac agaaggaaac ttcttatcga agtgacactc 2520
   tatatctaga aataagtacg atcttgggat atcgaatcta ggtatttttt tagcgaaaca 2580
   gttacgtgga tcgtcacaat gataacatcc attgttaatc tttgtcaaat attgctcgtc 2640
   caacgagtaa catccgtctg gagatatccc gttagaaata taaaaccaac taatattgag 2700
   aaattcatcc atggtggcat tttgtatgct gcgtttcttt ggctcttcta tcaaccacat 2760
   atctgcgacg gagcattttc tatctttaat atctagatta taacttattg tctcgtcaat 2820
   gtctatagtt ctcatctttc ccaacggcct cgcattaaat ggaggaggag acaatgactg 2880
   atatatttcg tccgtcacta cgtaataaaa gtaatgagga aatcgtataa atacggtctc 2940
   accatttcga catctggatt tcagatataa aaatctgttt tcaccgtgac tttcaaacca 3000
   attaatgcac cgaacatcca t                                           3021
<210> 8
   <211> 1908
   <212> DNA
   <213> Vaccinia virus
<400> 8
   ttataatttt accatctgac tcatggattc attaatatct ttataagagc tactaacgta 60
   taattcttta taactgaact gagatatata caccggatct atggtttcca taattgagta 120
   aatgaatgct cggcaataac taatggcaaa tgtatagaac aacgaaatta tactagagtt 180
   gttaaagtta atattttcta tgagctgttc caataaatta tttgttgtga ctgcgttcaa 240
   gtcataaatc atcttgatac tatccagtaa accgttttta agttctggaa tattattatc 300
   ccattgtaaa gcccctaatt cgactatcga atatcctgct ctgatagcag tttcaatatc 360
   gacggacgtc aatactgtaa taaaggtggt agtattgtca tcatcgtgat aaactactgg 420
   aatatggtcg ttagtaggta cggtaacttt acacaacgcg atatataact ttccttttgt 480
   accattttta acgtagttgg gacgtcctgc agggtattgt tttgaagaaa tgatatcgag 540
   aacagatttg atacgatatt tgttggattc ctgattattt actataatat aatctagaca 600
   gatagatgat tcgataaata gagaaggtat atcgttggta ggataataca tccccattcc 660
   agtattctcg gatactctat taatgacact agttaagaac atgtcttcta ttctagaaaa 720
   cgaaaacatc ctacatggac tcattaaaac ttctaacgct cctgattgtg tctcgaatgc 780
   ctcgtacaag gatttcaagg atgccataga ttctttgacc aacgatttag aattgcgttt 840
   agcatctgat ttttttatta aatcgaatgg tcggctctct ggtttgctac cccaatgata 900
   acaatagtct tgtaaagata aaccgcaaga aaatttatac gcatccatcc aaataaccct 960
   agcaccatcg gatgatatta atgtattatt atagattttc catccacaat tattgggcca 1020
   gtatactgtt agcaacggta tatcgaatag attactcatg taacctacta gaatgatagt 1080
   tcgtgtacta gtcataatat ctttaatcca atctaagaaa tttaaaatta gattttttac 1140
   actgttaaag ttaacaaagg tattacccgg gtacgtggat atcatatatg gtattggtcc 1200
   attatcagta atagctccat aaactgatac ggcgatggtt tttatatgtg tttgatctaa 1260
   cgaggaagaa attcgcaccc acaattcatc tctagatatg tatttaatat caaacggtaa 1320
   cacatcaatt tcgggacgcg tatatgtttc taaattttta atccaaatat aatgatgacc 1380
   tatatgccct attatcatac tgtcaactat agtacaccta gagaacttac gatacatctg 1440
   tttcctataa tcgttaaatt ttacaaatct ataacatgct aaaccttttg acgacaacca 1500
   ttcattaatt tctgatatgg aatctgtatt ctcgataccg tattgttcta aagccagtgc 1560
   tatatctccc tgttcgtggg aacgctttcg tataatatcg atcaacggat aatctgaagt 1620
   ttttggagaa taatatgact catgatctat ttcgtccata aacaatctag acataggaat 1680
   tggaggcgat gatcttaatt ttgtgcaatg agtcgtcaat cctataactt ctaatcttgt 1740
   aatattcatc atcgacataa tactatctat gttatcatcg tatattagta taccatgacc 1800
   ttcttcattt cgtgccaaaa tgatatacag tcttaaatag ttacgcaata tctcaatagt 1860
   ttcataattg ttagctgttt tcatcaagat ttgtaccctg tttaacat             1908
<210> 9
   <211> 1656
   <212> DNA <213> Vaccinia virus
<400> 9
   ttagttatta tctcccataa tcttggtaat acttacccct tgatcgtaag ataccttata 60
   caggtcatta catacaacta ccaattgttt ttgtacataa tagattggat ggttgacatc 120
   catggtggaa taaactactc gaacagatag tttatctttc cccctagata cattggccgt 180
   aatagttgtc ggcctaaaga atatctttgg tgtaaagtta aaagttaggg ttcttgttcc 240
   attattgctt tttgtcagta gttcattata aattctcgag atgggtccgt tctctgaata 300
   tagaacatca tttccaaatc taacttctag tctagaaata atatcggtct tattcttaaa 360
   atctattccc ttgatgaagg gatcgttaat gaacaaatcc ttggcctttg attcggctga 420
   tctattatct ccgttataga cgttacgttg actagtccaa agacttacag gaatagatgt 480
   atcgatgatg ttgatactat gtgatatgtg agcaaagatt gttctcttag tggcatcact 540
   atatgttcca gtaatggcgg aaaacttttt agaaatgtta tatataaaag aattttttcg 600
   tgttccaaac attagcagat tagtatgaag ataaacactc atattatcag gaacattatc 660
   aatttttaca tacacatcag catcttgaat agaaacgata ccatcttctg gaacctctac 720
   gatctcggca gactccggat aaccagtcgg tgggccatca ctaacaataa ctagatcatc 780
   caacaatcta ctcacatatg catctatata atctttttca tcttgtgagt accctggata 840
   cgaaataaat ttattatccg tatttccata ataaggttta gtataaacag agagcgatgt 900
   tgccgcatga acttcagtta cagtcgccgt tggttggttt atttgaccta ttactctcct 960
   aggtttctct ataaacgatg gtttaatttg tacattctta accatatatc caataaagct 1020
   caattcagga acataaacaa attctttgtt gaacgtttca aagtcgaacg aagagtcacg 1080
   aataacgata tcggatactg gattgaaggt taccgttacg gtaatttttg aatcggatag 1140
   tttaagactg ctgaatgtat cttccacatc aaacggagtt ttaatataaa cgtatactgt 1200
   agatggttct ttaatagtgt cattaggagt taggccaata gaaatatcat taagttcact 1260
   agaatatcca gagtgtttca aagcaattgt attattgata caattattat ataattcttc 1320
   gccctcaatt tcccaaataa caccgttaca cgaagagata gatacgtgat taatacattt 1380
   atatccaaca tatggtacgt aaccgaatct tcccatacct ttaacttctg gaagttccaa 1440
   actcagaacc aaatgattaa gcgcagtaat atactgatcc ctaatttcga agctagcgat 1500
   agcctgattg tctggaccat cgtttgtcat aactccggat agagaaatat attgcggcat 1560
   atataaagtt ggaatttgac tatcgactgc gaagacatta gaccgtttaa tagagtcatc 1620
   cccaccgatc aaagaattaa tgatagtatt attcat                           1656
<210> 10
<211> 915
<212> DNA
<213> Vaccinia virus
<400> 10
   ctagttttgt ttttctcgcg aatatcgtcg actcataaga aagagaatag cggtaagtat 60
   aaacacgaat actatggcaa taattgcgaa tgttttattc ccttcgatat atttttgata 120
   atatgaaaaa catgtctctc tcaaatcgga caaccatctc ataaaatagt tctcgcgcgc 180
   tggagaggta gttgctgctc gtataatctc cccagaataa tatacttgcg tgtcgtcgtt 240
   caatttatac ggatttctat agttctctgt tatataatac ggttttccat catgattaga 300
   cgacgacaat agtgttctaa atttagatag ttgatcagaa tgaatgttta ttggcgttgg 360
   aaaaattatc catacagcgt ctgcagagtg cttgatagtt gttcctagat atgtaaaata 420
   atccaacgta ctaggtagca aattgtctag ataaaatact gaatcaaacg gcgcagacgt 480
   attagcggat ctaatggaat ccaattgatt gactatcttt tgaaaatata catttttatg 540
   atccgatact tgtaagaata tagaaataat gataagtcca tcatcgtgtt tttttgcctc 600
   ttcataagaa ctatattttt tcttattcca atgaacaaga ttaatctctc cagagtattt 660
   gtacacatct atcaagtgat tggatccata atcgtcttcc tttccccaat atatatgtag 720
   tgatgataac acatattcat tggggagaaa ccctccactt atatatcctc ctttaaaatt 780
   aatccttact agttttccag tgttctggat agtggttggt ttcgactcat tataatgtat 840
   gtctaacggc ttcaatcgcg cgttagaaat tgctttttta gtttctatat taataggaga 900
   tagttgttgc ggcat                                                  915
<210> 11
   <211> 939
   <212> DNA
   <213> Vaccinia virus
<400> 11
   ttattcagca ttacttgata tagtaatatt aggcacagtc aaacattcaa ccactctcga 60
   tacattaact ctctcatttt ctttaacaaa ttctgcaata tcttcgtaaa aagattcttg 120
   aaacttttta gaatatctat cgactctaga tgaaatagcg ttcgtcaaca tactatgttt 180
   tgtatacata aaggcgccca ttttaacagt ttctagtgac aaaatgctag cgatcctagg 240
   atcctttaga atcacataga ttgacgattc gtctctctta gtaactctag taaaataatc 300
   atacaatcta gtacgcgaaa taatattatc cttgacttga ggagatctaa acaatctagt 360
   tttgagaaca tcgataagtt catcgggaat gacatacata ctatctttaa tagaactctt 420
   ttcatccagt tgaatggatt cgtccttaac caactgatta atgagatctt ctattttatc 480
   attttccaga tgatatgtat gtccattaaa gttaaattgt gtagcgcttc tttttagtct 540
   agcagccaat actttaacat cactaatatc gatatacaaa ggagatgatt tatctatggt 600
   attaagaatt cgtttttcga catccgtcaa aaccaattcc tttttgcctg tatcatccag 660
   ttttccatcc tttgtaaaga aattattttc tactagacta ttaataagac tgataaggat 720
   tcctccataa ttgcacaatc caaacttttt cacaaaacta gactttacga gatctacagg 780
   aatgcgtact tcaggtttct tagcttgtga ttttttcttt tgcggacatt ttcttgtgac 840
   caactcatct accatttcat tgattttagc agtgaaataa gctttcaatg cacgggcact 900
   gatactattg aaaacgagtt gatcttcaaa ttccgccat                        939
<210> 12
<211> 429
<212> DNA
<213> Vaccinia virus
<400> 12
   ttaattgcaa agatctatat aatcattata gcgttgactt atggactctg gaatcttaga 60
   cgatgtacag tcatctataa tcatggcata tttaatacat tgttttatag catagtagtt 120
   atctacgatg ttagatattt ctctcaatga atcaatcaca taatctaatg taggtttatg 180
   acataatagc attttcagca gttcaatgtt tctagattcg ttgatggcaa tggctataca 240
   tgtatatccg ttatttgatc taatgttgac atctgaaccg gattctagca gtaaagatac 300
   tagagattgt ttattatatc taacagcctt gtgaagaagt gtttctcctc gtttgtcaat 360
   catgttaatg tctttaagat aaggtaggca aatgtttata gtactaagaa ttgggcaagc 420
   ataagacat                                                         429
<210> 13
<211> 1038
<212> DNA
<213> Vaccinia virus
<400> 13
   atggatatct tcagggaaat cgcatcttct atgaaaggag agaatgtatt catttctcca 60
   gcgtcaatct cgtcagtatt gacaatactg tattatggag ctaatggatc cactgctgaa 120
   cagctatcaa aatatgtaga aaaggaggag aacatggata aggttagcgc tcaaaatatc 180
   tcattcaaat ccataaataa agtatatggg cgatattctg ccgtgtttaa agattccttt 240
   ttgagaaaaa ttggcgataa gtttcaaact gttgacttca ctgattgtcg cactatagat 300
   gcaatcaaca agtgtgtaga tatctttact gaggggaaaa tcaatccact attggatgaa 360
   ccattgtctc ctgatacctg tctcctagca attagtgccg tatactttaa agcaaaatgg 420
   ttgacgccat tcgaaaagga atttaccagt gattatccct tttacgtatc tccgacggaa 480
   atggtagatg taagtatgat gtctatgtac ggcaaggcat ttaatcacgc atctgtaaag 540
   gaatcattcg gcaacttttc aatcatagaa ctgccatatg ttggagatac tagtatgatg 600
   gtcattcttc cagacaagat tgatggatta gaatccatag aacaaaatct aacagataca 660
   aattttaaga aatggtgtaa ctctctggaa gctacgttta tcgatgttca cattcccaag 720
   tttaaggtaa caggctcgta taatctggtg gatactctag taaagtcagg actgacagag 780
   gtgttcggtt caactggaga ttatagcaat atgtgtaatt cagatgtgag tgtcgacgct 840
   atgatccaca aaacgtatat agatgtcaat gaagagtata cagaagcagc tgcagcaact 900
   tgtgcactgg tgtcagactg tgcatcaaca attacaaatg agttctgtgt agatcatccg 960
   ttcatctatg tgattaggca tgttgatgga aaaattcttt tcgttggtag atattgctct 1020
   ccgacaacta attgttaa 1038
<210> 14
   <211> 453
   <212> DNA <213> Vaccinia virus
<400> 14
   ttaatccatg gactcataat ctctatacgg gattaacgga tgttctatat acggggatga 60
   gtagttctct tctttaactt tatacttttt actaatcata tttagactga tgtatgggta 120
   atagtgtttg aagagctcgt tctcatcatc agaataaatc aatatctctg tttttttgtt 180
   atacagatgt attacagcct catatattac gtaatagaac gtgtcatcta ccttattaac 240
   tttcaccgca tagttgtttg caaatacggt taatcctttg acctcgtcga tttccgacca 300
   atctgggcgt ataatgaatc taaactttaa tttcttgtaa tcattcgaaa taatttttag 360
   tttgcatccg tagttatccc ctttatgtaa ctgtaaattt ctcaacgcga tatctccatt 420
   aataatgatg tcgaattcgt gctgtatacc cat 453
<210> 15
<211> 660
<212> DNA
<213> Vaccinia virus
<400> 15
   atggcgatgt tttacgcaca cgctctcggt gggtacgacg agaatcttca tgcctttcct 60
   ggaatatcat cgactgttgc caatgatgtc aggaaatatt ctgttgtgtc agtttataat 120
   aacaagtatg acattgtaaa agacaaatat atgtggtgtt acagtcaggt gaacaagaga 180
   tatattggag cactgctgcc tatgtttgag tgcaatgaat atctacaaat tggagatccg 240
   atccatgatc aagaaggaaa tcaaatctct atcatcacat atcgccacaa aaactactat 300
   gctctaagcg gaatcgggta cgagagtcta gacttgtgtt tggaaggagt agggattcat 360
   catcacgtac ttgaaacagg aaacgctgta tatggaaaag ttcaacatga ttattctact 420
   atcaaagaga aggccaaaga aatgagtaca cttagtccag gacctataat tgattaccac 480
   gtctggatag gagattgtat ctgtcaagtt actgctgtgg acgtacatgg aaaggaaatt 540
   atgagaatga gattcaaaaa gggtgcggtg cttccgatcc caaatctggt aaaagttaaa 600
   cttggggaga atgatacaga aaatctttct tctactatat cggcggcacc atcgaggtaa 660
<210> 16
<211> 957
<212> DNA
<213> Vaccinia virus
<400> 16
   atgacgaccg taccagtgac ggatatacaa aacgatttaa ttacagagtt ttcagaagat 60
   aattatccat ctaacaaaaa ttatgaaata actcttcgtc aaatgtctat tctaactcac 120
   gttaacaacg tggtagatag agaacataat gccgccgtag tgtcatctcc agaggaaata 180
   tcctcacaac ttaatgaaga tctatttcca gatgatgatt ctccggccac tattatcgaa 240
   agagtacaac ctcatactac tattattgac gatactccac ctcctacgtt tcgtagagag 300
   ttattgatat cggaacaacg tcaacaacga gaaaaaagat ttaatattac agtatcgaaa 360
   aatgctgaag caataatgga atctagatct atgatatctt ctatgccaac acaaacacca 420
   tccttgggag tagtttatga taaagataaa agaattcaga tgttggagga tgaagtggtt 480
   aatcttagaa atcaacgatc taatacaaaa tcatctgata atttagataa ttttaccaga 540
   atactatttg gtaagactcc gtataaatca acagaagtta ataagcgtat agccatcgtt 600
   aattatgcaa atttgaacgg gtctccctta tcagtcgagg acttggatgt ttgttcagag 660
   gatgaaatag atagaatcta taaaacgatt aaacaatatc acgaaagtag aaaacgaaaa 720
   attatcgtca ctaacgtgat tattattgtc ataaacatta tcgagcaagc attgctaaaa 780
   ctcggatttg aagaaatcaa aggactgagt accgatatca cttcagaaat tatcgatgtg 840
   gagatcggag atgactgcga tgctgtagca tctaaactag gaatcggtaa cagtccggtt 900
   cttaatattg tattgtttat actcaagata ttcgttaaac gaattaaaat tatttaa    957
<210> 17
   <211> 273
   <212> DNA
   <213> Vaccinia virus
<400> 17
   ttagttcatg gaaatatcgc tatgattggt atgaatgact ccgctaactc tgtggggtgc 60
   gcagtgcttt ccccacatag aataaattag cattccgact gtgataataa taccaagtat 120
   aaacgccata atactcaata ctttccatgt acgagtggga ctggtagact tactaaagtc 180
   aataaaggcg aagatacacg aaagaatcaa aagaatgatt ccagcgatta gcacgccgga 240
   aaaataattt ccaatcataa gcatcatgtc cat                               273
<210> 18
   <211> 3861
   <212> DNA
   <213> Vaccinia virus
<400> 18
   atggctgtaa tctctaaggt tacgtatagt ctatatgatc aaaaagagat taatgctaca 60
   gatattatca ttagtcatgt taaaaatgac gacgatatcg gtaccgttaa agatggtaga 120
   ctaggtgcta tggatggggc attatgtaag acttgtggga aaacggaatt ggaatgtttc 180
   ggtcactggg gtaaagtaag tatttataaa actcatatag ttaagcctga atttatttca 240
   gaaattattc gtttactgaa tcatatatgt attcactgcg gattattgcg ttcacgagaa 300
   ccgtattccg acgatattaa cctaaaagag ttatcgggac acgctcttag gagattaaag 360
   gataaaatat tatccaagaa aaagtcatgt tggaacagcg aatgtatgca accgtatcaa 420
   aaaattactt tttcaaagaa aaaggtttgt ttcgtcaaca agttggatga tattaacgtt 480
   cctaattctc tcatctatca aaagttaatt tctattcatg aaaagttttg gccattatta 540
   gaaattcatc aatatccagc taacttattt tatacagact actttcccat ccctccgctg 600
   attattagac cggctattag tttttggata gatagtatac ccaaagagac caatgaatta 660
   acttacttat taggtatgat cgttaagaat tgtaacttga atgctgatga acaggttatc 720
   cagaaggcgg taatagaata cgatgatatt aaaattattt ctaataacac ttccagtatc 780
   aatttatcat atattacatc cggcaaaaat aatatgatta gaagttatat tgtcgcccga 840
   cgaaaagatc agaccgctag atctgtaatt ggtcccagta catctatcac cgttaatgag 900
   gtaggaatgc ccgcatatat tagaaataca cttacagaaa agatatttgt taatgccttt 960
   acagtggata aagttaaaca actattagcg tcaaaccaag ttaaatttta ctttaataaa 1020
   cgattaaacc aattaacaag aatacgccaa ggaaagttta ttaaaaataa aatacattta 1080
   ttgcctggtg attgggtaga agtagctgtt caagaatata caagtattat ttttggaaga 1140
   cagccgtctc tacatagata caacgtcatc gcttcatcta tcagagctac cgaaggagat 1200
   actatcaaaa tatctcccgg aattgccaac tctcaaaatg ctgatttcga cggagatgaa 1260
   gaatggatga tattggagca aaatcctaaa gccgtaattg aacaaagtat tcttatgtat 1320
   ccgacgacgt tactcaaaca cgatattcat ggagcccccg tttatggatc tattcaagat 1380
   gaaatcgtag cagcgtattc attgtttaga atacaagatc tttgtttaga tgaagtattg 1440
   aacatcttgg ggaaatatgg aagaaagttc gatcctaaag gtaaatgtaa attcagcggt 1500
   aaagatatct atacttactt gataggtgaa aagattaatt atccgggtct cttaaaggat 1560
   ggtgaaatta ttgcaaacga cgtagatagt aattttgttg tggctatgag gcatctgtca 1620
   ttggctggac tcttatccga tcataagtcg aacgtggaag gtatcaactt tattatcaag 1680
   tcatcttatg tttttaagag atatctatct atttacggtt ttggggtgac attcaaagat 1740
   ctgagaccaa attcgacgtt cactaataaa ttggaggcca tcaacgtaga aaaaatagaa 1800
   cttatcaaag aagcatacgc caaatatctc aacgatgtaa gagacgggaa aatagttcca 1860
   ttatctaaag ctttagaggc ggactatgtg gaatccatgt tatccaactt gacaaatctt 1920
   aatatccgag agatagaaga acatatgaga caaacgctga tagatgatcc agataataac 1980
   ctcctgaaaa tggccaaagc gggttataaa gtaaatccca cagaactaat gtatattcta 2040
   ggtacttatg gacaacagag gattgatggt gaaccagcag agactcgagt attgggtaga 2100
   gtcttacctt actatcttcc agactctaag gatccagaag gaagaggtta tattcttaat 2160
   tctttaacaa aaggattaac aggttctcaa tattactttt cgatgctggt tgccagatct 2220
   caatctactg atatcgtctg tgaaacatca cgtaccggaa cactggctag aaaaatcatt 2280
   aaaaagatgg aggatatggt ggtcgacgga tacggacaag tagttatagg taatacgctc 2340
   atcaagtacg ccgccaatta taccaaaatt ctaggctcag tatgtaaacc tgtagatctt 2400
   atctatccag atgagtccat gacttggtat ttggaaatta gtgctctgtg gaataaaata 2460
   aaacagggat tcgtttactc tcagaaacag aaacttgcaa aaaagacatt ggcgccgttt 2520
   aatttcctag tattcgtcaa acccaccact gaggataatg ctattaaggt taaggatctg 2580
   tacgatatga ttcataacgt cattgatgat gtgagagaga aatacttctt tacggtatct 2640
   aatatagatt ttatggagta tatattcttg acgcatctta atccttctag aattagaatt 2700
   acaaaagaaa cggctatcac tatctttgaa aagttctatg aaaaactcaa ttatactcta 2760
   ggtggtggaa ctcctattgg aattatttct gcacaggtat tgtctgagaa gtttacacaa 2820
   caagccctgt ccagttttca cactactgaa aaaagtggtg ccgtcaaaca aaaacttggt 2880
   ttcaacgagt ttaataacct gactaatttg agtaagaata agaccgaaat tatcactctg 2940
   gtatccgatg atatctctaa acttcaatct gttaagatta atttcgaatt tgtatgtttg 3000
   ggagaattaa atccaaacat cactcttcga aaagaaacag ataggtatgt agtagatata 3060
   atagtcaata gattatacat caagagagca gaaattaccg aattagtcgt cgaatatatg 3120
   attgaacgat ttatctcctt tagcgtcatt gtaaaggaat ggggtatgga gacattcatt 3180
   gaggacgagg ataatattag atttactgtc tacctaaatt tcgttgaacc ggaagaattg 3240
   aatcttagta agtttatgat ggttcttccg ggtgccgcca acaagggcaa gattagtaaa 3300
   ttcaagattc ctatctctga ctatacggga tatgacgact tcaatcaaac aaaaaagctc 3360
   aataagatga ctgtagaact catgaatcta aaagaattgg gttctttcga tttggaaaac 3420
   gtcaacgtgt atcctggagt atggaataca tacgatatct tcggtatcga ggccgctcgt 3480
   gaatacttgt gcgaagccat gttaaacacc tatggagaag ggttcgatta tctgtatcag 3540
   ccttgtgatc ttctcgctag tttactatgt gctagttacg aaccagaatc agtgaataaa 3600
   ttcaagttcg gcgcagctag tactcttaag agagctacgt tcggagacaa taaagcattg 3660
   ttaaacgcgg ctcttcataa aaagtcagaa cctattaacg ataatagtag ctgccacttt 3720
   tttagcaagg tccctaatat aggaactgga tattacaaat actttatcga cttgggtctt 3780
   ctcatgagaa tggaaaggaa actatctgat aagatatctt ctcaaaagat caaggaaatg 3840
   gaagaaacag aagactttta a                                           3861
<210> 19
   <211> 2001
   <212> DNA
   <213> Vaccinia virus
<400> 19
   ttaacgagtt ccatttatat catccaatat tattgaaatg acgttgatgg acagatgata 60
   caaataagaa ggtacggtac ctttgtccac catctcctcc aattcatgct ctattttgtc 120
   attaacttta atgtatgaaa acagtacgcc acatgcttcc atgacagtgt gtaacacttt 180
   ggatacaaaa tgtttgacat tagtataatt gtttaagact gtcaatctat aatagatagt 240
   agctataata tattctatga tggtattgaa gaagatgaca atcttggcat attgatcatt 300
   taacacagac atggtatcaa cagatagctt gaatgaaaga gaatcagtaa ttggaataag 360
   cgtcttctcg atggagtgtc cgtataccaa catgtctgat attttgatgt attccattaa 420
   attatttagt tttttctttt tattctcgtt aaacagcatt tctgtcaacg gaccccaaca 480
   tcgttgaccg attaagtttt gattgatttt tccgtgtaag gcgtatctag tcagatcgta 540
   tagcctatcc aataatccat catctgtgcg tagatcacat cgtacacttt ttaattctct 600
   atagaagagc gacagacatc tggaacaatt acagacagca atttctttat tctctacaga 660
   tgtaagatac ttgaagacat tcctatgatg atgcagaatt ttggataaca cggtattgat 720
   ggtatctgtt accataattc ctttgatggc tgatagtgtc agagcacaag atttccaatc 780
   tttgacaatt tttagcacca ttatctttgt tttgatatct atatcagaca gcatggtgcg 840
   tctgacaaca cagggattaa gacggaaaga tgaaatgatt ctctcaacat cttcaatgga 900
   taccttgcta ttttttctgg cattatctat atgtgcgaga atatcctcta gagaatcagt 960
   atcctttttg atgatagtgg atctcaatga catgggacgt ctaaaccttc ttattctatc 1020
   accagattgc atggtgattt gtcttctttc ttttatcata atgtaatctc taaattcatc 1080
   ggcaaattgt ctatatctaa aatcataata tgagatgttt acctctacaa atatctgttc 1140
   gtccaatgtt agagtatcta catcagtttt gtattccaaa ttaaacatgg caacggattt 1200
   aattttatat tcctctatta agtcctcgtc gataataaca gaatgtagat aatcatttaa 1260
   tccatcgtac atggttggaa gatgcttgtt gacaaaatct ttaattgtct tgatgaaggt 1320
   gggactatat ctaacatctt gattaataaa atttataaca ttgtccatag gatactttgt 1380
   aactagtttt atacacatct cttcatcggt aagtttagac agaatatcgt gaacaggtgg 1440
   tatattatat tcatcagata tacgaagaac aatgtccaaa tctatattgt ttaatatatt 1500
   atatagatgt agtgtagctc ctacaggaat atctttaact aagtcaatga tttcatcaac 1560
   cgttagatct attttaaagt taatcatata ggcattgatt tttaaaaggt atgtagcctt 1620
   gactacattc tcattaatta accattccaa gtcactgtgt gtaagaagat tatattctat 1680
   cataagcttg actacatttg gtcccgatac cattaaagaa ttcttatgat ataaggaaac 1740
   agcttttagg tactcatcta ctctacaaga attttggaga gccttaacga tatcagtgac 1800
   gtttattatt tcaggaggaa aaaacctaac attgagaatg tcggagttaa tagcttccag 1860
   atacagtgat tttggcaata gtccgtgtaa tccataatcc agtaacacga gctggtgctt 1920
   gctagacacc ttttcaatgt ttaatttttt tgaaataagc tttgataaag ccttcctcgc 1980
   aaattccgga tacatgaaca t                                          2001
<210> 20
   <211> 1539
   <212> DNA <213> Vaccinia virus
<400> 20
   ctattgtaga aattgttttt cacagttgct caaaaacgat ggcagtgact tatgagttac 60
   gttacacttt ggagtctcat ctttagtaaa catatcataa tattcgatat tacgagttga 120
   catatcgaac aaattccaag tatttgattt tggataatat tcgtattttg catctgctat 180
   aattaagata taatcaccgc aagaacacac gaacatcttt cctacatggt taaagtacat 240
   gtacaattct atccatttgt cttccttaac tatatatttg tatagataat tacgagtctc 300
   gtgagtaatt ccagtaatta catagatgtc gccgtcgtac tctacagcat aaactatact 360
   atgatgtcta ggcatgggag acttttttat ccaacgattt ttagtgaaac attccacatc 420
   gtttaatact acatattttt catacgtggt ataaactcca cccattacat atatatcatc 480
   gtttacgaat accgacgcgc ctgaatatct aggagtaatt aagtttggaa gtcttatcca 540
   tttcgaagtg ccgtgtttca aatattctgc cacacccgtt gaaatagaaa attctaatcc 600
   tcctattaca tataactttc catcgttaac acaagtacta acttctgatt ttaacgacga 660
   catattagta accgttttcc attttttcgt ttcaagatct acccgcgata cggaataaac 720
   atgtctattg ttaatcatgc cgccaataat gtatagacaa ttatgtaaaa catttgcatt 780
   atagaattgt ctatctgtat taccgactat cgtccaatat tctgtcctag gagagtaatg 840
   ggttattgtg gatatataat cagagttttt aatgactact atattatgtt ttataccatt 900
   tcgtgtcact ggctttgtag atttggatat agttaatccc aacaatgata tagcattgcg 960
   catagtatta gtcataaact tgggatgtaa aatgttgatg atatctacat cgtttggatt 1020
   tttatgtatc cactttaata atatcatagc tgtaacatcc tcatgattta cgttaacgtc 1080
   ttcgtgggat aagatagttg tcagttcatc ctttgataat tttccaaatt ctggatcgga 1140
   tgtcaccgca gtaatattgt tgattatttc tgacatcgac gcattatata gttttttaat 1200
   tccatatctt ttagaaaagt taaacatcct tatacaattt gtggaattaa tattatgaat 1260
   catagttttt acacatagat ctactacagg cggaacatca attattacgg cagcaactag 1320
   tatcatttct acattgttta tggtgatgtt tatcttcttc cagcgcatat agtctaatag 1380
   cgattcaaac gcgtgatagt ttataccatt caatataatc gcttcatcct ttagatggtg 1440
   atcctgaatg cgtttaaaaa aattatacgg agacgccgta ataatttcct tattcacttg 1500
   tataatttcc ccattgatag aaaatatcac gctttccat 1539
<210> 21
<211> 2358
<212> DNA
<213> Vaccinia virus
<400> 21
   atggatgcgg ctattagagg taatgatgtt atctttgtcc ttaagactat aggtgtccca 60
   tcagcatgta gacaaaatga agatccaaga ttcgtagaag catttaaatg cgacgagtta 120
   aaaagatata ttgataataa tccagaatgt acactattcg aaagtcttag ggatgaggaa 180
   gcatactcta tagtcagaat tttcatggat gtagatttag acgcgtgtct agacgaaata 240
   gattatttaa cggctattca agattttatt atcgaggtgt caaactgtgt agctagattc 300
   gcgtttacag aatgcggtgc cattcatgaa aatgtaataa aatccatgag atctaatttt 360
   tcattgacta agtctacaaa tagagataaa acaagttttc atattatctt tttagacacg 420
   tataccacta tggatacatt gatagctatg aaacgaacac tattagaatt aagtagatca 480
   tctgaaaatc cactaacaag atcgatagac actgccgtat ataggagaaa aacaactctt 540
   cgggttgtag gtactaggaa aaatccaaat tgcgacacta ttcatgtaat gcaaccaccg 600
   catgataata tagaagatta cctattcact tacgtggata tgaacaacaa tagttattac 660
   ttttctctac aacgacgatt ggaggattta gttcctgata agttatggga accagggttt 720
   atttcattcg aagacgctat aaaaagagtt tcaaaaatat tcattaattc tataataaac 780
   tttaatgatc tcgatgaaaa taattttaca acggtaccac tggtcataga ttacgtaaca 840
   ccttgtgcat tatgtaaaaa acgatcgcat aaacatccgc atcaactatc gttggaaaat 900
   ggtgctatta gaatttacaa aactggtaat ccacatagtt gtaaagttaa aattgttccg 960
   ttggatggta ataaactgtt taatattgca caaagaattt tagacactaa ctctgtttta 1020
   ttaaccgaac gaggagacta tatagtttgg attaataatt catggaaatt taacagcgaa 1080
   gaacccttga taacaaaact aattctgtca ataagacatc aactacctaa ggaatattca 1140
   agcgaattac tctgtccgag gaaacgaaag actgtagaag ctaacatacg agacatgtta 1200
   gtagattcag tagagaccga tacctatccg gataaacttc cgtttaaaaa tggtgtattg 1260
   gacctggtag acggaatgtt ttactctgga gatgatgcta aaaaatatac gtgtactgta 1320
   tcaaccggat ttaaatttga cgatacaaag ttcgtcgaag acagtccaga aatggaagag 1380
   ttaatgaata tcattaacga tatccaacca ttaacggatg aaaataagaa aaatagagag 1440
   ctatatgaaa aaacattatc tagttgttta tgtggtgcta ccaaaggatg tttaacattc 1500
   ttttttggag aaactgcaac tggaaagtcg acaaccaaac gtttgttaaa gtctgctatc 1560
   ggtgacctgt ttgttgagac gggtcaaaca attttaacag atgtattgga taaaggacct 1620
   aatccattta tcgctaacat gcatttgaaa agatctgtat tctgtagcga actacctgat 1680
   tttgcctgta gtggatcaaa gaaaattaga tctgataata ttaaaaagtt gacagaacct 1740
   tgtgtcattg gaagaccgtg tttctccaat aaaattaata atagaaacca tgctacaatc 1800
   attatcgata ctaattacaa acctgtcttt gataggatag ataacgcatt aatgagaaga 1860
   attgccgtcg tgcgattcag aacacacttt tctcaacctt ctggtagaga ggctgctgaa 1920
   aataatgacg cgtacgataa agtcaaacta ttagacgagg ggttagatgg taaaatacaa 1980
   aataatagat atagatttgc atttctatac ttgttggtga aatggtacaa aaaatatcat 2040
   gttcctatta tgaaactata tcctacaccc gaagagattc ctgactttgc attctatctc 2100
   aaaataggta ctctgttagt atctagctct gtaaagcata ttccattaat gacggacctc 2160
   tccaaaaagg gatatatatt gtacgataat gtggttactc ttccgttgac tactttccaa 2220
   cagaaaatat ccaagtattt taattctaga ctatttggac acgatataga gagcttcatc 2280
   aatagacata agaaatttgc caatgttagt gatgaatatc tgcaatatat attcatagag 2340
   gatatttcat ctccgtaa                                                 2358
<210> 22
   <211> 612
   <212> DNA
   <213> Vaccinia virus
<400> 22
   ttaataatcg tcagtattta aactgttaaa tgttggtata tcaacatcta ccttatttcc 60
   cgcagtataa ggtttgttgc aggtatactg ttcaggaatg gttacattta tacttcttct 120
   atagtcctgt ctttcgatgt tcatcacata tgcaaagaac agaataaaca aaataatgta 180
   agaaataata ttaaatatct gtgaattcgt aaatacattg attgccataa taattacagc 240
   agctacaata cacacaatag acattcccac agtgttgcca ttacctccac gatacatttg 300
   agttactaag caataggtaa taactaagct agtaagaggc aatagaaaag atgagataaa 360
   tatcatcaat atagagatta gaggagggct atatagagcc aagacgaaca aaatcaaacc 420
   gagtaacgtt ctaacatcat tatttttgaa gattcccaaa taatcattca ttcctccata 480
   atcgttttgc atcatacctc catctttagg cataaacgat tgctgctgtt cctctgtaaa 540
   taaatcttta tcaagcactc cagcacccgc agagaagtcg tcaagcatat tgtaatatct 600
   taaataactc at                                                       612